# EUROPÄISCHE PATENTSCHRIFT

(11) **EP 1 604 007 B1**
(45) Veröffentlichungstag und Bekanntmachung des Hinweises auf die Patenterteilung: **16.11.2016**
(21) Anmeldenummer: 03782475.2
(22) Anmeldetag: 22.12.2003
(51) Int. Cl.: C12M 1/00

(54) **VORRICHTUNG UND VERFAHREN ZUR PARALLELEN, AUTOMATISIERTEN KULTIVIERUNG VON ZELLEN UNTER TECHNISCHEN BEDINGUNGEN**
DEVICE AND METHOD FOR PARALLEL, AUTOMATED CULTIVATION OF CELLS IN TECHNICAL CONDITIONS
DISPOSITIF ET PROCEDE DE MISE EN CULTURE PARALLELE AUTOMATISEE DE CELLULES DANS DES CONDITIONS TECHNIQUES

(30) Priorität: 23.12.2002 DE 10260691
(43) Veröffentlichungstag der Anmeldung: 14.12.2005
(73) Patentinhaber: Technische Universität München, 80333 München (DE)
(72) Erfinder: PUSKEILER, Robert, 80797 München (DE); WEUSTER-BOTZ, Dirk, 85221 Dachau (DE); ZACHER, Karl-Heinz, 85656 Buch am Buchrain (DE)
(74) Vertreter: Pfenning, Meinig & Partner mbB
(86) Internationale Anmeldenummer: PCT/EP2003/014752
(87) Internationale Veröffentlichungsnummer: WO 2004/058935

(56) Entgegenhaltungen:
- EP-A- 1 046 707
- WO-A-97/09353
- DE-A- 3 902 162
- GB-A- 1 408 306
- US-A- 3 066 921
- US-A- 3 764 836
- US-A- 4 611 790
- US-A- 5 075 234
- US-A- 5 744 351
- US-B1- 6 464 387
- DATABASE WPI Week 199332 Derwent Publications Ltd., London, GB; AN 1993-257051 XP002331685 -& SU 1 755 747 A (PLANTS PHYSIOLOGY INST) 23. August 1992 (1992-08-23)
- PATENT ABSTRACTS OF JAPAN Bd. 16, Nr. 168, 22. April 1992 (1992-04-22) -& JP 04 016226 A (YANO YOSHIO), 21. Januar 1992 (1992-01-21)
- PATENT ABSTRACTS OF JAPAN Bd. 5, Nr. 16, 30. Januar 1981 (1981-01-30) -& JP 55 142535 A (KURIMOTO IRON WORKSLTD), 7. November 1980 (1980-11-07)

## Beschreibung

Die vorliegende Erfindung betrifft eine Vorrichtung zur Kultivierung von Zellen, eine Anordnung derartiger Vorrichtungen, ein hierfür geeignetes Rührorgan sowie ein Kulturverfahren für Zellen. Derartige Vorrichtungen und Verfahren werden zur Kultivierung von Zellen im Milliliter-Maßstab benötigt. Diese werden besonders für parallele Ansätze bei der Stamm- bzw. Bioprozessentwicklung in der chemischen Industrie, z.B. für die Reaktionsoptimierung bzw. Katalysatoroptimierung, im Bereich des Umweltschutzes, für die Optimierung von Abwasserbehandlungen bzw. chemischen oder biologischen Behandlung von Feststoffen bzw. von Abluft, oder im Bereich der Lebensmitteltechnologie eingesetzt.

Die DE 39 02 162 A1 betrifft einen Apparat zur Kultivierung von Mikroorganismen im Litermaßstab, wobei das Kulturmedium mit Schaufeln durchmischt wird, welche in einem Neigungswinkel zur horizontalen Fläche ausgeführt sind. Im oberen Teil des Gehäuses des Apparates befinden sich Eintrittsstutzen für die Einführung von Nährmedium und Impfstoff. Im unteren Teil des Gehäuses befindet sich außerdem ein Austrittsstutzen für die Ableitung des fertigen Produkts und ein Belüfter.

Bislang wurden als Reaktoren zur Kultivierung von Zellen in Flüssigkeitssäulen im Millilitermaßstab, wie das insbesondere für Parallelreaktionen zur Erprobung bestimmter biotechnologischer Techniken erforderlich ist, Schüttelkolben oder Rührkessel eingesetzt.

Der klassische Parallelreaktor in der Biotechnologie ist der Schüttelkolben, mit dem seit dem letzten Jahrhundert einfache Satzversuche im Parallelansatz manuell durchgeführt werden. Schüttelkolben werden auf Schütteltablaren montiert, in Inkubatoren bei vorgegebener Temperatur mit einer bestimmten Schüttelfrequenz in eine Rotationsbewegung mit gegebener Exzentrizität versetzt. Durch die Bewegung des Reaktionsgefäßes erfolgt eine Durchmischung der im Reaktionsgefäß enthaltenen Flüssigkeit, in der die biochemische Reaktion stattfindet.

Über Oberflächenbegasung wird der für viele biochemische Reaktionen notwendige Sauerstoff aus der Gasphase in die Flüssigphase eingetragen. Hohe Sauerstoffeinträge sind folglich nur möglich, wenn ein sehr großes Oberflächen/Volumen Verhältnis eingestellt wird. Das bedeutet, dass sehr große Schüttelkolben (1-2 l Volumen) mit sehr wenig Reaktionsmedium (10-20 ml) bei möglichst großer Exzentrizität und Schüttelfrequenz (400 rpm) betrieben werden müssen. Unter diesen Bedingungen werden Sauerstoffübergangskoeffizienten k_{L}a von bis zu 0.07 s⁻¹ erreicht.

Der Leistungseintrag in einen Schüttelkolben erfolgt durch die Reibung der Flüssigkeit an der Innenwand des rotierenden Reaktionsgefäßes. Es erfolgt damit eine relativ gleichmäßige Energiedissipation.

Die Vorteile der Schüttelkolbenkultivierung sind die einfache Handhabung und der relativ geringe apparative Aufwand.

Alternativ hierzu können auch Rührkesselreaktoren eingesetzt werden. Wesentliche reaktionstechnische Unterschiede zwischen dem Reaktionsgefäß Schüttelkolben und dem Reäktionsgefäß Rührkesselreaktor - dem klassischen Produktionsreaktor der Biotechnologie - sind der geringere Sauerstoffeintrag, das weitaus geringere Verhältnis der maximalen lokalen Energiedissipation zum mittleren Leistungseintrag und die fehlende Kontrolle wichtiger Prozessgrößen (wie beispielsweise pH oder P_{O2}). Dies führt dazu, dass Reaktionsverläufe in den meisten Fällen nicht direkt vom Reaktionssystem Schüttelkolben in das Reaktionssystem Rührkesselreaktor übertragen werden können und damit in der Bioprozessentwicklung zusätzliche personal- und zeitintensive sequentielle Experimente in Laborbioreaktoren erforderlich sind.

Technische Ansätze dieses Problem zu lösen, sind die Bereitstellung von parallelen Rührkesseleinheiten mit vollständiger individueller Mess- und Regeltechnik. Kommerziell erhältlich sind Parallelreaktorsysteme mit 4 oder 6 Rührkesselreaktoren mit einem Volumen von bis zu 0,5 l. Die gewonnenen Prozessdaten sind meist gut auf größere Rührkesselreaktoren übertragbar. Der Kapital-, Personal- und Zeitaufwand wird jedoch außerordentlich hoch, wenn mehrere dieser Parallelreaktoreinheiten zur Bioprozessentwicklung eingesetzt werden müssen.

Eine neuere Entwicklung ist der Betrieb von parallelen Kleinreaktoren in einem Inkubator mit intermittierender Substratdosierung und paralleler pH-Kontrolle (DE 197 09 603 A1). Als parallele Kleinreaktoren werden entweder Blasensäulen oder gerührte Säulen mit einem Arbeitsvolumen von 200 ml eingesetzt (DE 195 29 099 A1). Damit können Sauerstoffübergangskoeffizienten und volumenspezifische Leistungseinträge wie im klassischen Rührkesselreaktor erzielt werden. Die Anzahl paralleler Bioreaktoren ist jedoch beschränkt (≤ 16). Eine weitere Parallelisierung ist auf Basis dieser Technologieplattform praktisch nicht möglich. Zur simultanen Konzentrationsmessung von Schlüsselkomponenten des Reaktionsmediums (Substrat- oder Produktkonzentration) werden parallele Probenahme- und Analysensysteme verwendet (EP 0995 098 A1).

Eine weitere, einfache Möglichkeit, noch weitaus mehr Reaktoren parallel zu betreiben, ist die Verwendung von Mikrotiterplatten in Inkubationsschüttlern zur Satz-Kultivierung von Zellen. Mikrotiterplatten mit 24, 48, 96 oder mehr Vertiefungen zur Kultivierung von Zellen weisen jedoch in noch stärkerem Maße die selben reaktionstechnischen Restriktionen wie Schüttelkolben auf. Zusätzlich erweist sich die Verdunstung in diesem Maßstab als problematisch, da der relative Verdunstungsvolumenstrom bezogen auf das Ausgangsvolumen durch das sehr große Oberflächen/Volumen-Verhältnis und dem geringen Reaktionsvolumen (≤ 1,5 ml) sehr viel größer ist als beispielsweise im Schüttelkolben oder Rührkesselreaktor.

Um eine zügige Umsetzung neuer biologischer Erkenntnisse in technisch realisierbare und wirtschaftliche Verfahren durchführen zu können, muß die bisher sequentielle Vorgehensweise mit einfachen Parallelansätzen im Schüttelkolben und der nachfolgenden Optimierung der Reaktionsbedingungen im kontrollierten Laborrührkesselreaktor überwunden werden. Dies ist nur möglich, wenn eine möglichst große Zahl von Rührkesselreaktoren automatisiert unter technischen, kontrollierten Reaktionsbedingungen im Parallelansatz durchgeführt werden kann.

Um eine Parallelisierung von Rührkesselreaktoren zu ermöglichen, müssen diese so einfach wie möglich aufgebaut und möglichst ohne Einbauten zu betreiben sein. Ideale Reaktionsgefäße sind beispielsweise sterilisierbare Reagenzgläser oder Mikrotiterplatten mit entsprechend großen Kavitäten.

Der Leistungseintrag kann in diesem Maßstab einfach durch Magnetrührantriebe erfolgen. In technischen Rührreaktoren wird der Sauerstoffeintrag in das Reaktionsmedium bei Volumenbegasung primär durch den Leistungseintrag des Rührorgans und sekundär durch die Gasleerrohrgeschwindigkeit bestimmt. Eine Primärdispergierung der Gasphase wie sie im klassischen Rührkesselreaktor über einen Gasverteiler am Reaktorboden erfolgt, ist in parallel angeordneten Milliliter-Rührkesselreaktoren jedoch nur sehr aufwendig zu realisieren: Die Reaktionsgefäße müssten mit einer individuellen Gaszufuhr und einem Gasverteiler versehen werden. Der Gasverteiler müsste sicherstellen, dassss in jedem der parallelen Reaktionsgefäße exakt die gewünschte Gasleerrohrgeschwindigkeit erzielt wird.

Bisher ist es nur möglich, entweder eine hohe Zahl von einfachen, unkontrollierten Parallelreaktionen unter nichttechnischen Bedingungen in Mikrotiterplatten oder in Schüttelkolben durchzuführen oder eine relativ kleine Anzahl von Bioreaktoren unter kontrollierten, technischen Bedingungen zu betreiben.

Da es erforderlich ist, einen hohen Gaseintrag in die Reaktionsgefäße zu erzielen, bietet es sich an, die Sterilgaszufuhr von oben in die Gefäße durchzuführen. Hierzu wäre die einfachste sterile Abgrenzung der Einsatz eines Sterilfilters als Abdeckung der einzelnen Reaktoren bzw. einer gesamten Anordnung von Reaktoren. Ein solches Sterilfilter müsste neben der mechanischen Barriere für Kontaminanten jedoch eine gute Gastransporteigenschaft besitzen, um eine Sauerstofflimitierung in den Reaktionsgefäßen zu vermeiden.

Darüber hinaus stellt der Deckel des Reaktionsgefäßes die einzige Möglichkeit zum Eingriff in den Reaktionsablauf dar, beispielsweise zur Zugabe von Substrat, Titrationsmitteln oder Induktoren während der Reaktion, zur Probenentnahme zur Prozesskontrolle oder zum Einbringen von Messsonden. Um diese Eingriffe steriltechnisch einwandfrei durchführen zu können, wird das Sterilfilter gewöhnlich als Septum ausgeführt. Die Gasdurchlässigkeit von Septen, die meist auf Siliconbasis beruhen, ist jedoch unzureichend, so dass beide Funktionen nicht durch ein Material erfüllt werden können. Problematisch ist es daher, einen einfachen und gesichert sterilen Zugang zu dem bzw. den Reaktionsgefäßen zu haben, wobei dieser Zugang jedoch unter allen Umständen steril sein soll.

Aufgabe der vorliegenden Erfindung ist es daher, eine Vorrichtung zur Kultivierung von Zellen in Flüssigkeitssäulen im Millilitermaßstab zu schaffen, mit der zum einen ein hoher Gas- und Leistungseintrag erzielt wird und zum andern einzelne oder auch eine große Zahl von derartigen Vorrichtungen parallelisiert effektiv betrieben werden können. Weiterhin ist es Aufgabe der vorliegenden Erfindung, eine derartige Anordnung paralleler Vorrichtungen zur Verfügung zu stellen sowie ein Rührorgan, mit dem der gewünschte hohe Leistungs- und Gaseintrag erzielt werden kann. Die Aufgabe der vorliegenden Erfindung ist es zusätzlich, entsprechende Kulturverfahren von Zellen in Flüssigkeitssäulen im Millilitermaßstab zur Verfügung zu stellen.

Diese Aufgabe wird durch die Vorrichtung nach Anspruch 1, das Rührorgan nach Anspruch 10, das Rührorgan nach Anspruch 1, die Anordnung nach Anspruch 30 sowie das Verfahren nach Anspruch 48 gelöst. Vorteilhafte Weiterbildungen der jeweiligen Vorrichtung, Anordnung, Rührorgan bzw. des erfindungsgemäßen Verfahrens werden in den jeweiligen abhängigen Ansprüchen gegeben.

Mit der erfindungsgemäßen Lösung, ist es möglich, einzelne oder auch eine Vielzahl von automatisierten Rührkesselreaktoren, beispielsweise 24,48 oder 96 oder mehr Rührkesselreaktoren sowohl für die Stamm- als auch für die Bioprozessentwicklung zeiteffektiv unter technischen Reaktionsbedingungen zu betreiben. Damit ist die parallele automatisierte Kultivierung von Zellen im Millilitermaßstab unter individuell kontrollierten Reaktionsbedingungen, wie Temperatur, Wasserstoffionenaktivität, Sauerstoffeintrag, Leistungseintrag sowie Medienzufuhr möglich, so dass Reaktionsverläufe, wie sie im klassischen Rührkesselbioreaktor erzielt werden, im Parallelansatz durchführbar sind. Unter Millimetermaßstab wird dabei vorteilhafterweise ein Bereich für das gerührte Flüssigkeitsvolumen von 0,5 bis 50 ml, vorzugsweise von 1 bis 30 ml, vorzugsweise von 5 bis 20 ml verstanden. Mit Hilfe der erfindungsgemäßen Vorrichtung und des erfindungsgemäßen Verfahrens ist eine direkte Übertragung der so gewonnenen (fed batch) Prozessverläufe aus dem erfindungsgemäßen Milliliter-Maßstab in den Liter-Maßstab (und umgekehrt) möglich. Die erfindungsgemäßen Milliliter-Rührreaktoren erlauben eine ebenso effiziente Sauerstoffversorgung von Organismen in Flüssigkultur wie Rührkesselreaktoren größeren Maßstabs mit Volumenbegasung.

Bei Einsatz der erfindungsgemäßen Rührkesselreaktoren ist eine Parallelisierung von einer großen Zahl von Bioreaktoren in einem Bioreaktorblock möglich, wobei deren Betrieb erstmalig durch Einsatz von Laborrobottern, beispielsweise Pipettier-Robotern und dergleichen automatisierbar ist und so ein effizienter, individuell kontrollierter Parallelbetrieb ermöglicht wird. Erst die erfindungsgemäße Vorrichtung und auch die erfindungsgemäße Abdeckung ermöglichen die Verwendung eines Laborroboters und damit einen Quantensprung in der Gewinnung relevanter Prozessdaten.

Werden die Laborroboter mit geeigneten Screening und Optimierroutinen betrieben, so kann eine Prozessoptimierung, beispielsweise bezüglich der Medienzusammensetzung, der Induktionsverfahren und der Dosierprofile, systematisch und mit hoher Zeiteffizienz erzielt werden. Die vollständige Digitalisierung der parallelen Prozessentwicklung ermöglicht weiterhin eine neuartige Datentransparenz und Datenverfügbarkeit.

Da die Volumina der Rührkesselreaktoren, mit denen dennoch eine aussagekräftige Information über den jeweiligen Prozessverlauf gewonnen werden kann, nunmehr stark minimiert werden können, beispielsweise statt 500 ml lediglich noch 5 ml, kann bei gleichem Gesamtvolumen durch Einsatz von 100x mehr Reaktionsgefäßen ein Vielfaches an Information gewonnen bzw. bei gleicher Informationsmenge der Zeitaufwand durch die realiesierte Automatisierung minimiert werden. Bei Einsatz geeigneter Versuchsplanungs-Algorithmen kann damit ein Quantensprung in der Effektivität der Bioprozessentwicklung ermöglicht werden.

Die vorliegende Erfindung beruht entscheidend darauf, dass erkannt wurde, dass der Gaseintrag von der Oberfläche der Flüssigkeitssäule in die Flüssigkeitssäule in einem Rührkesselreaktor dadurch verbessert wird, dass entweder der Behälter und/oder das Rührorgan derart ausgebildet werden, dass die Strömungsgeschwindigkeit sich längs einer Stromlinie oder Bahnlinie, die beim Rührkesselreaktor im Kreis verläuft, sich längs der Stromlinie bzw. Bahnlinie örtlich und/oder zeitlich ändert. Dies führt zu einem räumlich und/oder zeitlich pulsierenden Bernoulli-Effekt. Dies kann beispielsweise in einer zum Boden des Rührkesselreaktors (Behälters) gerichtete Förderung der Kultursuspension führen, welche zu intentensiven Eintrag von Glasblasen führt. Als Stromlinien werden dabei Linien in der Strömung bezeichnet, deren Richtung in jedem Rammpunkt mit der Richtung des Geschwindigkeitsektors übereinstimmt. Als Bahnlinie werden Linien bezeichnet, die von den Flüssigkeitsteilchen durchlaufen werden.

Basierend auf dieser Erkenntnis ist es nun möglich, entweder den Behälter (Rührkesselreaktor) und/oder das Rührorgan geeignet auszugestalten.

Zum einen ist es möglich, den Behälter selbst so auszugestalten, dass sein Innenvolumen keine rotationssymmetrische Form aufweist. Dort wo der Flüssigkeitsstrom sich dann verbreitert, befindet sich ein Bereich geringer Strömungsgeschwindigkeit und damit hohen Druckes während dort wo die Flüssigkeit zwischen dem Rührorgan und der Behälterwand durchströmt ein Bereich hoher Strömungsgeschwindigkeit und damit niedrigen Druckes vorliegt. Damit ist eine räumlich längs des Umfangs des Behälters variierende Strömungsgeschwindigkeit bzw. variierender Flüssigkeitsdruck gegeben.

Der selbe Effekt wird erzielt, wenn das Rührorgan innerhalb des beliebig oder auch rotationssymmetrisch geformten Behälters außermittig bzw. exzentrisch angeordnet wird. In diesem Falle ergeben sich wieder Abstände zwischen Rührorgan und Wand des Behälters, die längs des Umfangs des Behälters variieren und dadurch unterschiedliche Strömungsgeschwindigkeiten und Druckverhältnisse induzieren.

Eine weitere Möglichkeit um diesen pulsierenden Bernoulli-Effekt zu erzielen, ist es, längs des Umfangs des Behälters Strömungsbrecher anzuordnen. Unter Strömungsbecher werden dabei Elemente verstanden, die sich in der Strömung der gerührten Flüssigkeit befinden und so für diese einen Strömungswiderstand darstellen. Strömungsbrecher führen zu einer Verengung des Strömungsquerschnitts. Da der Abstand zwischen Rührer und Wand des Gefäßes größer ist als zwischen Rührer und Strömungsbrecher, bilden sich zwischen Rührer und Strömungsbrecher wieder Bereiche hoher Strömungsgeschwindigkeit und zwischen Rührer und freien Wandbereichen Bereiche geringer Strömungsgeschwindigkeit aus. Der Strömungsbrecher muss dabei nicht in der Drehebene des Rührorgans angeordnet sein, sondern kann unterhalb, in der Drehebene oder auch bzw. zusätzlich auch oberhalb der Drehebene des Rührorgans angeordnet sein. In all diesen Fällen ergibt sich ein pulsierender Bernoulli-Effekt. Die Strömungsbrecher können vorteilhafterweise einstückig mit der Lagerung des Rührorgans oder einstückig mit dem Behälter ausgebildet sein, beispielsweise im Spritzgussverfahren.

Die Spaltabstände sollen dabei so gewählt werden, dass sich ein ausreichender pulsierender Bernoulli-Effekt ergibt, jedoch die Scherkräfte nicht so große werden, dass die in der Suspension enthaltenen Zellen zerstört werden. Spaltabstände > 0,05 mm, vorzugsweise > 0,1 mm und/oder < 20 mm, vorzugsweise < 3 mm sind hierfür besonders geeignet. Als Behälter sind klassische Rührkolben, Reagenzgläser oder auch die Kavitäten einer Mikrotiterplatte oder einer speziell gefertigten Platte gleicher Kavitätenanordnung mit adäquaten Durchmessern geeignet.

Eine weitere Möglichkeit einen pulsierenden Bernoulli-Effekt zu erzeugen besteht darin, das Rührorgan geeignet auszubilden. Hierzu wird in das Rührorgan eine Bohrung eingebracht, die von der Unterseite und/oder Seitenwand des Rührorgans sich zu einer Seitenwand bzw. zur Oberseite des Rührorgans erstreckt. Vorteilhafterweise verläuft die Bohrung unter einem Winkel α mit 0° ≤ α < 90° zur Dreh- bzw. Mittelachse des Rührorgans, wobei sich dieser Winkel nach oben öffnet.

Vorteilhaft können auch weitere Durchgangskanäle mit einer entsprechenden Öffnung nach unten von der Oberseite und/oder Seitenwand des Rührorgans sich zu dessen Seitenwand bzw. Unterseite erstrecken.

Diese Kanäle können auch nur teilweise durch das Rührorgan unter dem Winkel α verlaufen und dann auf einen bezüglich der auf der Drehachse senkrecht stehenden Ebene in dieser Ebene oder unter einem Winkel < 90° nach oben oder unten zu dieser Ebene verknüpft verlaufenden weiteren Kanal stoßen und in diesen einmünden, der seinerseits an der seitlichen Außenwand des Rührorgans mit einer Öffnung endet.

Durch derartige Bohrungen wird ebenfalls eine Strömung vom Boden des Behälters zur Seitenwand des Rührorgans induziert, die zu einem veränderten Druck an den von der Drehachse abgewandten Öffnung der Bohrungen bzw. Kanäle führt und so einen pulsierenden Bernoulli-Effekt induziert.

Vorteilhafterweise weisen die Gefäße einen Verschluss bzw. Abdeckung auf, die das Gefäß bzw. eine Anordnung von Gefäßen steril abdeckt. In diese Verschlüsse können zum einen Gasverteilerstrukturen zur Zufuhr von Sterilgas eingebracht sein. Der Verschluss kann hierzu beispielsweise aus einer Doppelbodenplatte bestehen, wobei die Gasverteilerstruktur im Zwischenraum zwischen den beiden Platten der Doppelbodenplatte angeordnet sind. Der Verschluss kann auch aus einer oder mehreren Platten bestehen, wobei die Gasverteilerstrukturen an der Unterseite der untersten Platte angeordnet sind.

Vorteilhafterweise werden die Gasverteilerstrukturen derart angelegt, dass ausgehend von einer zentralen Gaszufuhr einzelne Kanäle als Abzweigungen zu den jeweiligen Behältern führen. Vorteilhafterweise werden die Kanäle dabei so geführt, dass sie sowohl gleiche Querschnitte, gleiche Länge als auch die gleiche Anzahl von Biegungen oder Knicken aufweisen. Dadurch wird ein gleichmäßiger Gasdruck auf sämtlichen Behältern bewirkt. Dabei können entweder lediglich die Abzweigungen in gleicher Weise angelegt sein oder auch das gesamte System.

Weiterhin weist der Verschluss vorteilhafterweise für jeden einzelnen Behälter eine Öffnung auf, die nach außen von dem Behälter zugeführtem Sterilgas durchströmt wird. Diese Öffnung kann beispielsweise ein Röhrchen sein, durch das eine Kanüle oder jede andere längliche Probenentnahme- oder Sensoreinheit eingeführt werden kann. Da dies dann im Gegenstrom erfolgt, ist es lediglich erforderlich, die jeweils eingebrachte Einheit zuvor zu sterilisieren und dann durch das Röhrchen in die Suspension einzuführen, um eine Kontamination des Reaktionsgefäßes zu vermeiden.

Weiterhin kann der Verschluss Stege aufweisen, die bei einer Anordnung von Behältern die einzelnen Behälter voneinander steril isoliert. Ein weiterer Steg, der ebenfalls dem jeweiligen Behälter zugeordnet ist, kann so ausgebildet sein, dass er in die Suspension eintaucht und dabei den Einlass für das Sterilgas von dem oben genannten Auslass abtrennt. Dies führt dazu, dass das Sterilgas zu einem Weg durch die Kultursuspension gezwungen wird und dadurch die Begasung der Kultursuspension weiter verbessert wird.

Im folgenden werden Beispiele erfindungsgemäßer Vorrichtung, Anordnung von Vorrichtung, Rührorgane und zugehöriger Verfahren beschrieben.

Es zeigen
- Fig. 1: einen Längsschnitt durch eine Anordnung (Bioreaktorblock) von Bioreaktoren;
- Fig. 2: verschiedene Varianten der Anordnung von Strömungsbrechern und Rührorganen in einem Bioreaktor;
- Fig. 3: die Anordnung eines erfindungsgemäßen Rührorgans und Strömungsbrecher in einem Bioreaktor;
- Fign. 4 bis 6: weitere erfindungsgemäße Vorrichtungen;
- Fig. 7: ein erfindungsgemäßes Rührorgan;
- Fign. 8 bis 13: weitere erfindungsgemäße Rührorgane;
- Fig. 14: eine weitere Anordnung von Bioreaktoren und die Struktur des dazugehörigen Verschlusses;
- Fig. 15: die Struktur eines weiteren Verschlusses;
- Fig. 16: eine Gesamtanordnung mit Pipettier-Roboter;
- Fig. 17: den prinzipiellen Ablauf einer parallelen Steuerung einer Bioreaktorenanordnung;
- Fig. 18: maximale Sauerstoffübergangs-Koeffizienten für erfindungsgemäße Magnetrührorgane verschiedenen Typs;
- Fig. 19: Sauerstoffübergangskoeffizienten für Magnetrührorgane verschiedener erfindungsgemäßer Typen;
- Fign. 20 bis 22: die Ergebnisse der Kultivierung von Escherichia coli in erfindungsgemäßen Rührsystemen verschiedenen Typs;
- Fig. 23: eine Tabelle der für die Messungen gemäß der Figuren 18 bis 22 verwendeten Rührsysteme;
- Fign. 24 bis 26: weitere erfindungsgemäße Rührorgane;
- Fign. 27 bis 28: weitere erfindungsgemäße Vorrichtungen;
- Fig. 29: maximale Sauerstoffübergangskoeffizienten für ein Rührorgan gemäß Fig. 25;
- Fig. 30: Biomassetrockenkonzentrationen erzielt mit einem Rührorgan gemäß Fig. 25.

Im folgenden werden in sämtlichen Figuren für gleiche oder ähnliche Elemente gleiche oder ähnliche Bezugszeichen verwendet.

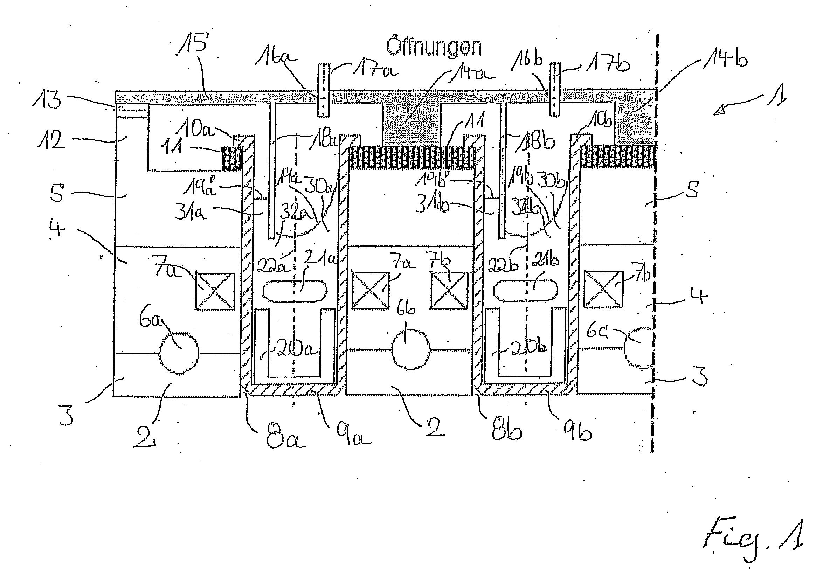

Fig. 1 zeigt eine erfindungsgemäße Anordnung von Reaktionsgefäßen 9a, 9b als Behälter in einem Modulsystem 2, das im folgenden insgesamt als Bioreaktorblock 1 bezeichnet wird.

Dieser Bioreaktorblock 1 enthält bis zu 96 Kavitäten bzw. Bohrungen 8a, 8b, wobei diese in verschiedenen Formaten angeordnet sein können, beispielsweise 4 x 3, 4 x 5, 8 x 3, 8 x 6 bzw. 8 x 12 Bohrungen. Die Durchmesser der Bohrungen 8a, 8b liegen vorteilhafterweise zwischen 10 und 35 mm. Sie werden so im Bioreaktorblock 1 angeordnet, dass entsprechend dimensionierte Kleinstreaktionsgefäße 9a, 9b in diese formschlüssig eingebracht werden können. Der Bioreaktorblock 1 ist dabei aus einer Vielzahl von horizontalen Schichten 3, 4, 5 aufgebaut, wobei die unterste Schicht 3 eine Basisplatte, die darüberliegende Schicht 4 einen Mittelteil und die darüberliegende Schicht 5 einen Oberteil bildet. Zwischen der Basisplatte 3 und dem Mittelteil 4 sind Bohrungen 6a, 6b, 6c angeordnet, die als Wärmetauscher von einem Fluid mit geeigneter Temperatur durchströmt werden und so den gesamten Bioreaktorblock 1 temperieren. Im Mittelteil 4 sind weiterhin die Bohrungen 8a, 8b umgebend magnetisch induktive Magnetantriebe 7a, 7b angeordnet wie sie beispielsweise aus der US 4 568 195 bekannt sind. Das Oberteil 5 enthält einen seitlich überstehenden Rand 12 längs der Außenseite des gesamten Biorektorblockes 1, in den eine Sterilgaszufuhr 13 zur Zufuhr von sterilem Gas von außen in den Bioreaktorblock 1 eingebracht ist.

Im Innenbereich des Bioreaktorblocks 1 kann auf das Oberteil 5 eine Distanzscheibe 11 aufgelegt werden. Da das einzelne Rührgefäß 9a, 9b an seiner Oberseite bzw. Oberkante einen ringförmigen Flansch 10a, 10b aufweist, stützt sich dieser Flansch 10a, 10b auf die Distanzscheibe 11. Durch Wahl einer geeignet dicken Distanzscheibe 11 kann die Höhe des Reaktorgefäßes 9a, 9b eingestellt werden. Damit ist dann die Rührhöhe eines in dem Gefäß 9a, 9b angeordneten Magnetrührers 21a, 21b über dem Boden des Reaktionsgefäßes 9a, 9b definiert. In das jeweilige Reaktionsgefäß 9a, 9b ist an dessen Boden aufliegend ein einstückiger Strömungsbrecher 20a, 20b angeordnet, der an zwei Stellen auf der Umfangslinie des Reaktorgefäßes 9a, 9b den Querschnitt des Reaktorgefäßes verengt. Der Strömungsbrecher 20a, 20b endet mit seiner Oberkante im vorliegenden Beispiel unterhalb der Rührebene des Rührorgans 21a, 21b. In anderen Beispielen kann sich jedoch der Strömungsbrecher auch seitlich neben das Rührorgan erstrecken oder dieses sogar nach oben überragen. Es ist auch möglich, dass die Strömungsbrecher nur oberhalb und/oder in der Rührebene des Rührorgans angeordnet sind.

Der auf den Bioreaktorblock 1 aufgebrachte Deckel bzw. Abdeckung 15 weist Mittelstege 14a, 14b auf, die sich bis zu der Distanzscheibe 11 erstrecken und so die einzelnen Reaktionsgefäße 9a, 9b als Trennwände steril voneinander isolieren. Weiterhin sind Stege 18a, 18b vorgesehen, die sich bis in den Reaktionsraum 9a, 9b erstrecken und diesen ebenfalls als Trennwände in zwei Kompartimente teilen. Zuletzt weist der Deckel 15 auch noch je eine Bohrung 16a, 16b auf, durch die sich jeweils ein Röhrchen 17a, 17b erstreckt. Dieses Röhrchen 17a, 17b stellt eine beständig offene Verbindung zwischen der Außenseite des Bioreaktorblocks 1 und jeweils einem der Reaktorgefäße 9a, 9b dar.

Wird nun eine Kultursuspension 30a, 30b in die jeweiligen Gefäße 9a, 9b eingebracht, so trennt der Steg 18a, 18b die Oberfläche 19a, 19b der Flüssigkeit 30a, 30b in zwei voneinander getrennte Bereiche 19a, 19a' bzw. 19b, 19b'. Wird nun der Rührer 21a bzw. 21b in Drehung zersetzt, so bildet sich eine konvexe Flüssigkeitsoberfläche 19a bzw. 19b aufgrund der Mitdrehung der Flüssigkeit aus. Für die Flüssigkeit 30a bzw. 30b an der Oberfläche 19a' bzw. 19b' ist dieser Effekt nicht so ausgeprägt und hier nicht weiter dargestellt.

Wird nun über die Sterilgaszufuhr 13 ein Gas auf die Oberfläche 19a' unter Überdruck geleitet, so muss dieses um den Behälter 9a wieder zu verlassen durch den linken Schenkel 31a der Flüssigkeitssäule unter dem Steg 18a hindurch in den rechten Schenkel 32a der Flüssigkeitssäule strömen und von dort das Gefäß über das Röhrchen 17a wieder verlassen. Da das Röhrchen 17a auf diese Art und Weise ständig von innen nach außen durchströmt wird, ist das Reaktionsgefäß 9a steril, obwohl das Röhrchen 17a offen ist und einen beständig offenen Zugang zu dem Gefäß 9a bildet. Dasselbe gilt für das Reaktionsgefäß 9b entsprechend.

Vorteilhafterweise können nun über das Röhrchen 17a bzw. 17b ohne weiteres Eingriffe in den Reaktionsablauf durchgeführt werden. Dies bedeutet beispielsweise, dass über das Röhrchen 17a, 17b Substrat oder Titrationsmittel oder Induktoren hinzugegeben werden können, dass Proben entnommen werden können oder Messsonden, beispielsweise-pH-Elektroden in die Flüssigkeit 30a bzw. 30b eingeführt werden können. Das Einführen entsprechender Sonden erfolgt dabei im Gegenstrom des ausströmenden sterilen Gases, so dass eine Kontamination des Gefäßes 9a bzw. 9b vermieden wird.

Die Abdeckung 15 realisiert daher eine Sterilabdeckung für den Bioreaktorblock 1 mit einer zentralen Gaseinspeisung 13 über ein Sterilfilter. Eine individuelle Verteilung des sterilen Gases über zu den einzelnen Milliliter-Rührreaktoren führende Gasverteilerstrukturen kann ebenfalls erfolgen.

Der konvektive Luftstrom durch das Röhrchen 17a, 17b, verhindert also im Betrieb den Eintrag von Fremdkeimen über die Umgebungsluft. Das offene Führungsrohr 17a, 17b ist hier beispielsweise aus Aluminium gefertigt und ist dadurch auch als Zugang mit sterilen Pipettenspitzen oder Anstechkanülen geeignet.

Werden Gasverteilerstrukturen in der Sterilabdeckung eingesetzt, so sind diese so zu gestalten, dass eine Kreuzkontamination durch Aerosolverschleppung oder Schaumbildung ausgeschlossen wird.

Der Bioreaktorblock 1 und die Abdeckung 15 sind so passgenau gestaltet, dass sie unter einer sterilen Arbeitsbank, gegebenenfalls nach steriler Befüllung der Einzelreaktoren 9a, 9b mit Reaktionsmedium 30a, 30b zu einer Funktionseinheit zusammengefügt werden können.

Die Sterilisation des Bioreaktorblocks 1 und der Abdeckung 15 kann entweder gemeinsam in einem Autoklaven oder auch als Einzelkomponenten erfolgen. Zur Sterilisation des Bioreaktorblocks 1 im Autoklaven ist eine kostenaufwendige Kapselung der induktiven Antriebe 7a, 7b erforderlich, um ein direktes Autoklavieren.zu ermöglichen.

Alternativ können auch wie hier vorgestellt, Bioreaktoreinsätze 9a, 9b verwendet werden (entsprechend zu Mikrotiterplatten) die getrennt vom Bioreaktorblock 1 sterilisierbar sind. Diese Bioreaktoreinsätze 9a, 9b können auch als sterile Einmal- bzw. Wegwerfartikel ausgestaltet sein, sofern deren Material und Produktionskosten gering sind.

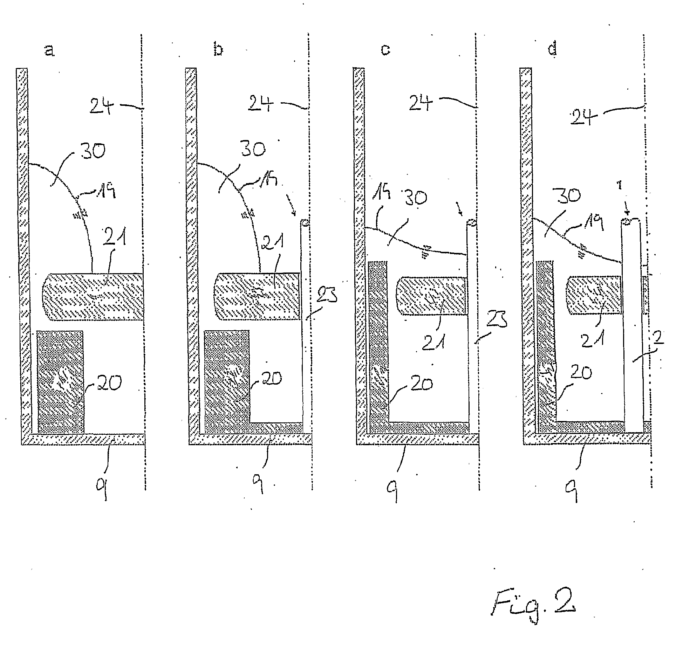

Fig. 2 zeigt in den Teilbildern a bis d die Verwendung von Strömungsbrechern 20 und Rührorganen 21 zur Erzeugung eines pulsierenden Bernoulli-Effektes. Dabei ist jeweils nur die linke Hälfte eines Reaktionsgefäßes 9 dargestellt.

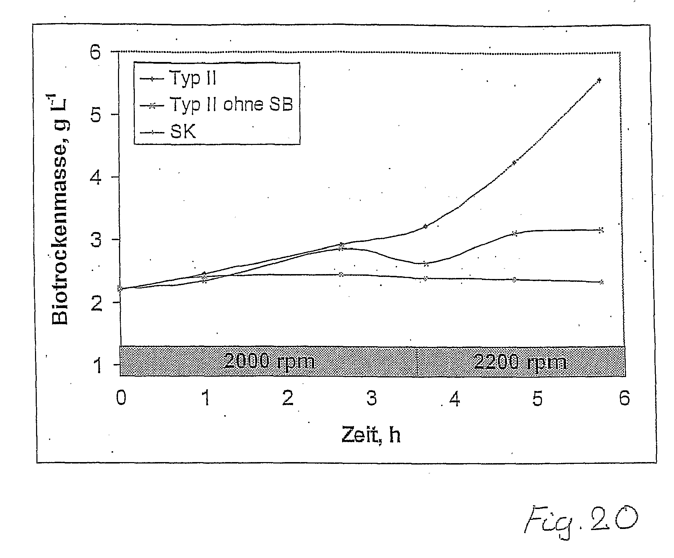

In den Figuren 20a und 20b ist die Oberkante des Strömungsbrechers 20 unterhalb der Rührebene des Rührorgans 21.

In Fig. 2a ist das Rührorgan mittig in dem Gefäß 9 gelagert, wobei die Lagerung magnetisch induktiv erfolgt.

In Fig. 2b ist das Magnetrührorgan 21 über eine Welle 23 gelagert, über die es auch angetrieben werden kann.

In den Figuren 2c und 2d erstreckt sich der Strömungsbrecher 20 seitlich über die Drehebene des Magnetrührorgans 21 hinaus.

In Fig. 2c ist das Rührorgan 21 so wie in Fig. 2b über eine Welle 23 gelagert und wird gegebenenfalls auch über diese oder magnetisch angetrieben. In Fig. 2d ist die Welle 23 innerhalb des Rührgefäßes 9 exzentrisch außerhalb der Mittelachse 24 des Gefäßes 9 gelagert, so dass der durch den Strömungsbrecher erzeugte pulsierende Bernoulli-Effekt hier noch weiter verstärkt wird.

In den einzelnen Fällen der Fign. 2a und 2d bildet sich damit eine unterschiedlich geformte Flüssigkeitsoberfläche 19 der Flüssigkeit 30 aus.

Bei sämtlichen Beispielen 2b bis 2d ist es möglich, sofern die Welle 23 nicht dem Antrieb des Magnetrührorgans 21 dient, die Welle 23 und den Strömungsbrecher 20 einstückig zu fertigen und als Einheit in das Reaktionsgefäß 9 passgenau einzuschieben.

Im folgenden werden nun Beispiele für erfindungsgemäße Rührorgane und erfindungsgemäße Reaktionsgefäße dargestellt. Denn bei Verwendung eines geeigneten Rührorgans besteht prinzipiell die Möglichkeit, auf eine Primärdispergierung der Gasphase durch einen Gasverteiler wie im Stand der Technik zu verzichten, da kleine Reaktionsgefäße im Vergleich zu Labor-Rührkesselreaktoren ein weitaus höheres Oberfläche/Volumen-Verhältnis aufweisen.

In der vorliegenden Erfindung wurden geeignete, vorteilhafterweise dampfsterilisierbare Magnetrührorgane entwickelt, die eine axiale Förderung von der Flüssigkeitsoberfläche zum Boden des Reaktionsgefäßes (Einsaugen der Gasphase) und eine effektive Dispergierung der Gasphase in möglichst kleine Gasblasen mit hoher Sauerstoffaustauschfläche (hohe lokale Energiedissipation) im Reaktionsmedium sowie eine Freisetzung der verbrauchten Gasblasen an der Flüssigkeitsoberfläche bewirken. Diese Magnetrührorgane besitzen einen Grundkörper, der vorteilhafterweise aus Teflon gefertigt sein kann und einen bis vier Magnetkerne (Ferrit oder seltene Erdmagnete wie z. B. SmCo(SanariumCobalt) oder NdFeB(NeodymEisenBor)) als Antriebsmittel enthält. Die im folgenden vorgestellten Magnetrührorgane haben vorteilhafterweise die folgenden Dimensionen und Formen:
- Kreiszylindrische Magnetrührorgane 3 bis 20 mm Durchmesser, Höhe von 2 bis 25 mm.
- Eiförmige Magnetrührorgane runder Zentralquerschnitt (Durchmesser 15 mm), Länge von 3 bis 20 mm.
- Quaderförmige Magnetrührorgane 3 x 8 mm bis 9 x 20 mm Grundfläche und Höhen von 4 bis 25 mm.

Diese Grundkörper sind vorteilhafterweise mit Bohrungen bzw. Kanälen versehen. Diese sind zwischen 3 und 20 mm lang und besitzen Durchmesser, die an die Größe des Rührorgans angepasst sein sollte, vorteilhafterweise von 0,5 bis 5 mm, vorteilhafterweise von 0,5 bis 3 mm. Hierbei sind unterschiedliche Anordnungen der Bohrungen realisierbar:
- Unter einem nach oben offenen Winkel α zur Vertikalen verlaufende Kanäle induzieren eine Ringströmung im Reaktionsgefäß.
- Unter einem nach oben offenen Winkel α zur Vertikalen verlaufende Kanäle treffen vor deren Austritt in die Flüssigkeit auf unter einem Winkel β zur Horizontalen verlaufende Luftkanäle.
- Vertikal verlaufende Kanäle mit einem Abstand von 0-3 mm begünstigen das Entgasen der Flüssigphase.

Diese Magnetrührorgane werden dabei auf Geschwindigkeiten bis zu 4000 U/min beschleunigt, beispielsweise durch ein geeignetes magnetisches Drehfeld oder durch eine Welle.

Das Einsaugen der Gasphase in die Reaktionsgefäße mit diesen Rührorganen setzt bei einer Mindestdrehzahl des Magnetrührorgans ein und wird durch Steigerung der Drehzahl stärker. Diese Mindestdrehzahl ist abhängig vom eingesetzten Magnetrührorgan, von der Position des Magnetrührorgans unterhalb der ruhenden Flüssigkeitsoberfläche und von den Stoffeigenschaften der Flüssigkeit.

Ein besonders effektives Einsaugen der Gasphase und Dispergierung in Gasblasen im Reaktionsmedium kann in Reaktionsgefäßen mit Strömungsbrechern erfolgen, die längs der Gefäßwand in der umlaufenden Flüssigkeitsströmung angeordnet sind. Diese vorteilhafterweise ein bis vier Strömungsbrecher können entweder unterhalb und/oder oberhalb oder über die gesamte Gefäßhöhe an der Gefäßwand angeordnet sein.

Das Magnetrührorgan wird bevorzugt selbstzentrierend in einem geeigneten, rotierenden Magnetfeld betrieben. Es kann jedoch wie in Fig. 2, gezeigt auch eine Befestigung des Magnetrührorgans auf einer im Reaktionsgefäß fixierten Welle erfolgen.

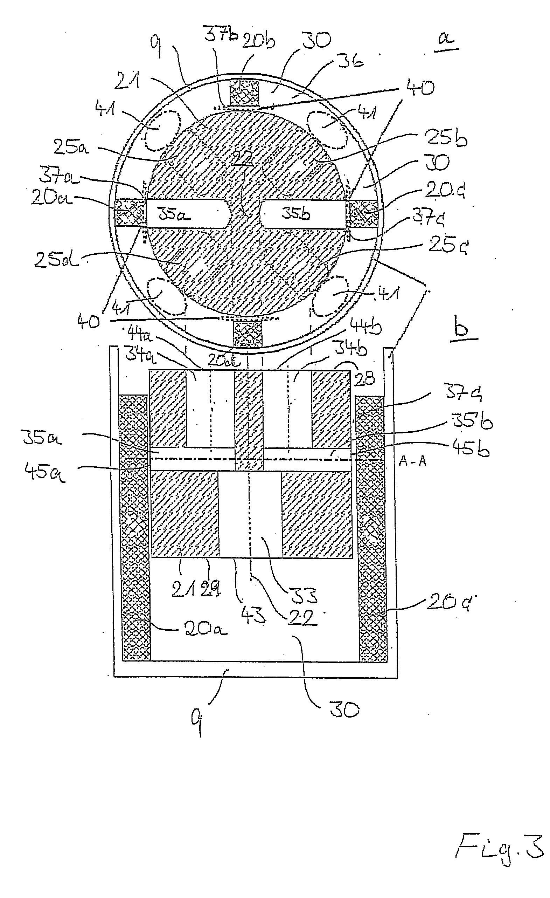

Die Fign. 3 bis 13 zeigen verschiedene Ausführungsformen erfindungsgemäßer Rührgefäße bzw. Rührorgane.

Fig. 3 zeigt in Fig. 3b ein Rührgefäß 9, bei dem an jeweils gegenüberliegenden Seiten insgesamt vier Strömungsbrecher 20a bis 20d angeordnet sind. Diese sind in 90° Abstand auf den Umfang des Rührgefäßes 9 verteilt und erstrecken sich bis in die Drehebene des in dem Gefäß 9 angeordneten Rührorgans 21. Das Rührorgan 21 dreht sich um seine Mittelachse 22 und ist zentrisch in dem Gefäß 9 angeordnet. Es weist ausgehend von seiner Unterseite 29 eine Bohrung 33 mit einer unteren Öffnung 43 auf, die sich in senkrechter Richtung, d. h. unter einem Winkel von 0° zur Drehachse 22 nach oben erstreckt. Von der Oberseite 28 des Drehorgans 21 erstrecken sich zwei Bohrungen 34a und 34b mit Öffnungen 44a und 44b an der Oberseite 28 des Drehorgans 21 nach unten. Sämtliche Bohrungen 33, 34a und 34b münden in horizontale Bohrungen 35a und 35b, die sich um 180° zueinander versetzt bis zur seitlichen Außenseite des Rührorgans 21 erstrecken und dort Öffnungen 45a und 45b bilden.

Wenn das Rührorgan 21 um seine Mittelachse 22 in einer Flüssigkeit 30 dreht, induziert der Kanal 33 eine Ringströmung im Reaktionsgefäß gemeinsam mit dem horizontal verlaufenden Kanal 35a. Die Kanäle 34a und 34b saugen ihrerseits Gas von oben ein und führen ebenfalls zu einer verbesserten Begasung der im Gefäß 9 befindlichen Flüssigkeit 30.

Fig. 3a zeigt einen Querschnitt längs der Linie A-A in Fig. 3b durch die gesamte Anordnung. Unter der Annahme, dass sich das Rührorgan 21 um die Achse 22 in Uhrzeigerrichtung dreht (diese Annahme wird auch bei den folgenden Figuren eingehalten) bilden sich nun zwischen den Brechern 20a bis 20d und dem Rührorgan 21 jeweils schmale Spalte 37a bis 37d mit Bereichen 40 mit hoher Strömungsgeschwindigkeit und damit niedrigem Flüssigkeitsdruck aus. Zwischen den Strömungsbrechern 20a bis 20d längs des Gefäßumfangs liegt ein großer Spalt 36 zwischen dem Rührorgan 21 und der Wand des Gefäßes 9 vor in dem dementsprechend Bereiche 41 mit geringer Strömungsgeschwindigkeit und damit großen hydrostatischem Druck auftreten. Insgesamt führt der Betrieb eines solchen Reaktionsgefäßes 9 zu einer örtlichen Schwankung der Strömungsgeschwindigkeit, d. h. zu einem örtlich pulsierenden Bernoulli-Effekt.

Durch die Bohrungen 33, 34a, 34b, 35a und 35b werden weiterhin zeitlich längs der Öffnungen 45a und 45b im Gefäß umlaufende, pulsierende Schwankungen der Strömungsgeschwindigkeit induziert, die ebenfalls zu einer Begasung des Reaktionsvolumens führen.

Der Antrieb des Rührorgans 21 erfolgt magnetisch induktiv über in das Rührorgan 21 eingelagerte Magnete 25a bis 25d.

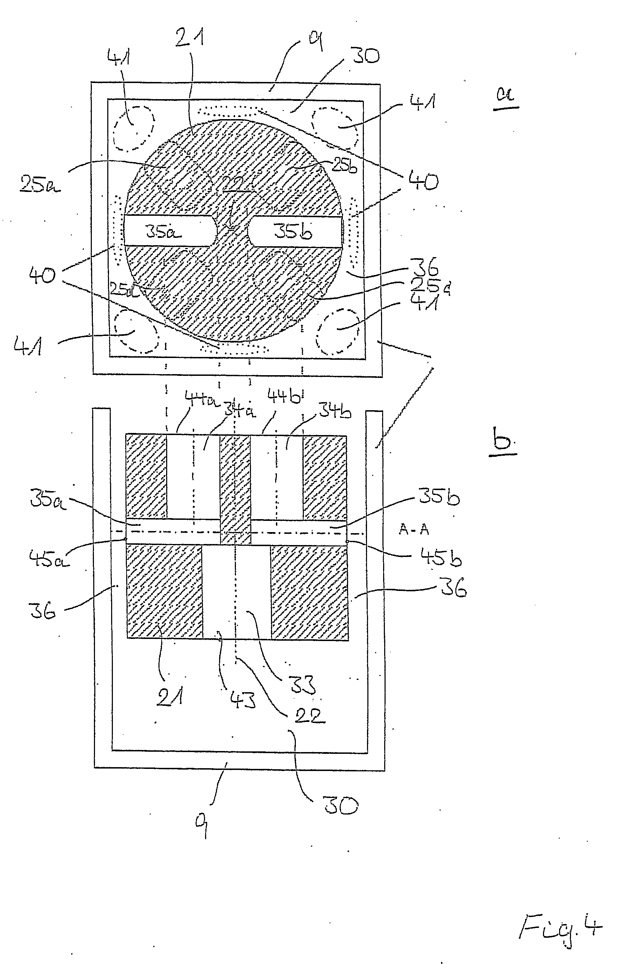

Fig. 4 zeigt eine Anordnung mit einem zu Fig. 3 identischen Rührorgan 21, wobei hier jedoch das Reaktionsgefäß wie in Fig. 4a, dem Schnitt längs der Linie A-A in Fig. 4b, zu erkennen ist, einen rechteckigen Querschnitt aufweist. Strömungsbrecher sind nicht vorhanden. In diesem-Falle ändert sich die Spaltbreite zwischen Rührorgan, das rotationssymmetrisch aufgebaut ist und der Wand des Rührgefäßes 9 räumlich periodisch, da die Spalten in den Ecken des Rührgefäßes 9 breiter sind als zwischen der Mitte der jeweiligen Wand des Rührgefäßes 9 und dem Rührorgan 21. Dies führt wiederum zu Bereichen 40 mit hoher Strömungsgeschwindigkeit in der Mitte der Wände und Bereichen 41 mit geringer Strömungsgeschwindigkeit in den Ecken des Gefäßes 9. Die Wirkung dieses so räumlich pulsierenden Bernoulli-Effektes wird verstärkt durch die Verwendung eines Rührorgans 21 mit den vorbeschriebenen Bohrungen, die einen zeitlich pulsierenden Bernoulli-Effekt induzieren.

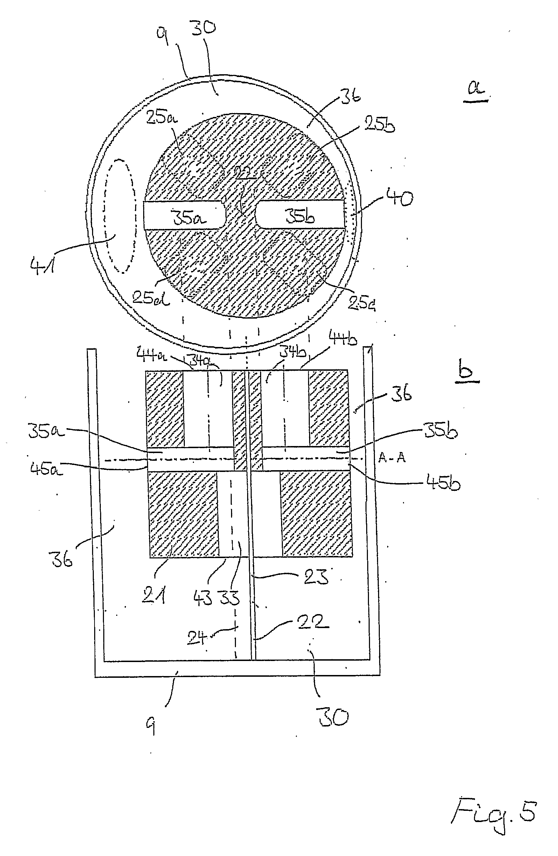

Fig. 5 zeigt wiederum dasselbe Rührorgan 21, das nunmehr jedoch in einem rotationssymmetrischen Rührgefäß 9 angeordnet ist. Allerdings erfolgt die Anordnung des Rührorgans 21 exzentrisch zur Mittelachse 24 des Gefäßes 9 auf einer Welle 23. Nunmehr ist durch die exzentrische Anordnung dafür gesorgt, dass der Spaltabstand 36 zwischen der Wand des Rührgefäßes 9 und dem Rührorgan 21 auf einer Seite des Gefäßes größer ist als auf der anderen Seite des Gefäßes. Dementsprechend bilden sich wieder Zonen 41 geringer Strömungsgeschwindigkeit 41 im Bereich des großen Spaltes und Zonen 40 hoher Strömungsgeschwindigkeit im Bereich des schmalen Spaltes aus. Auch hier wird der so mit einer Periode von 360° pulsierende Bernoulli-Effekt verstärkt durch die Bohrungen 33, 34a, 34b, 35a, 35b,... in dem Rührorgan 21.

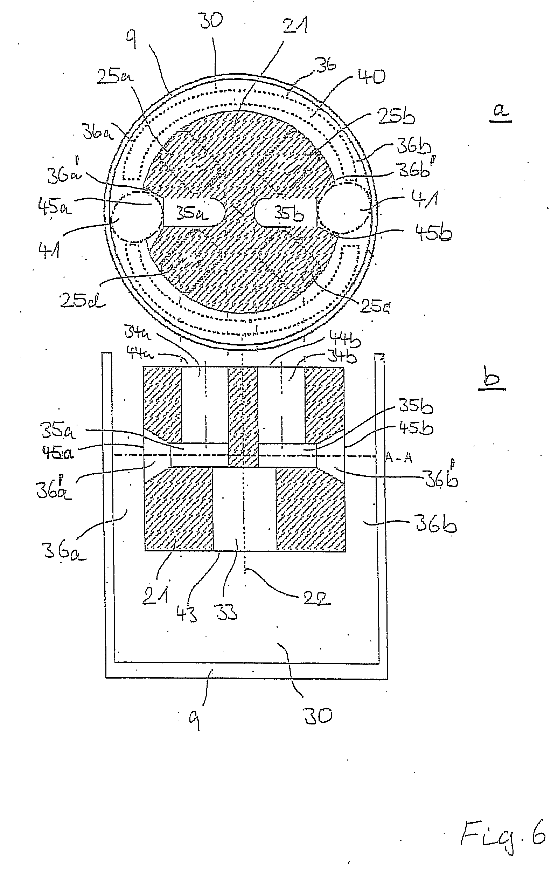

Auch in Fig. 6 wird ein ähnliches Rührorgan 21 verwendet wie in der vorigen Figur. In diesem Falle liegt wiederum ein zylinderförmiges Reaktionsgefäß 9 und ein zylinderförmiges Rührorgan 21 vor. Der Abstand 36 zwischen der Wand des Rührgefäßes 9 und dem Rührorgan 21 ist nun auf dem vollen Umfang nahezu identisch mit zwei Ausnahmen. Denn die horizontalen Bohrungen 35a und 35b sind beim vorliegenden Rührorgan 21 im Bereich ihrer seitlichen Öffnungen 45a und 45b konisch aufgeweitet. In diesen Bereichen liegt jeweils eine Erweiterung 36a' bzw. 36b' des Spaltes 36 vor, so dass dort Zonen 41 niedriger Strömungsgeschwindigkeit sich ausbilden. Daher laufen diese Zonen 41 niedriger Strömungsgeschwindigkeit nunmehr mit dem Rührorgan 21 innerhalb des Gefäßes 9 um. Dies führt zu einem örtlich und zeitlich pulsierenden Strömungsfeld und damit wiederum zu dem gewünschten pulsierenden Bernoulli-Effekt.

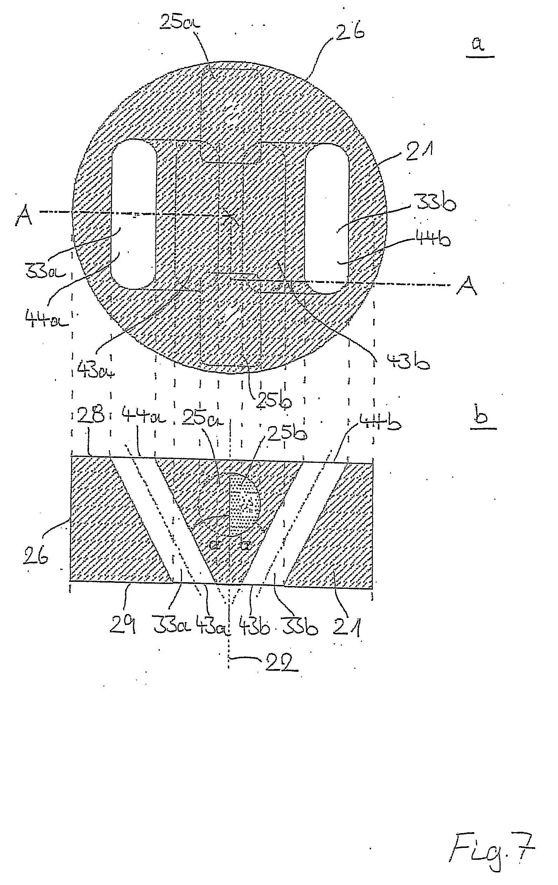

Fig. 7 zeigt die einfachste Form eines Rührorgans 21 mit schräg nach außen verlaufenden Bohrungen 33a und 33b. Die Bohrungen 33a, 33b beginnen an der Unterseite 29 des Rührorgans 21 mit einer Öffnung 43a, 43b und enden an der Oberseite 28 des Rührers 21 in einer Öffnung 44a bzw. 44b. Fig. 7b zeigt dabei einen Schnitt längs der Linie A-A aus Fig. 7a.

Die Bohrungen weisen dabei einen länglichen Querschnitt auf, wie in Fig. 7a in der Aufsicht zu erkennen ist. Sie führen zu einer Förderung von Flüssigkeit von dem Boden 29 des Rührorgans 21 und damit des Rührgefäßes 9 nach oben und tragen so zur Ausbildung der Flüssigkeitsoberfläche als Trombe bei.

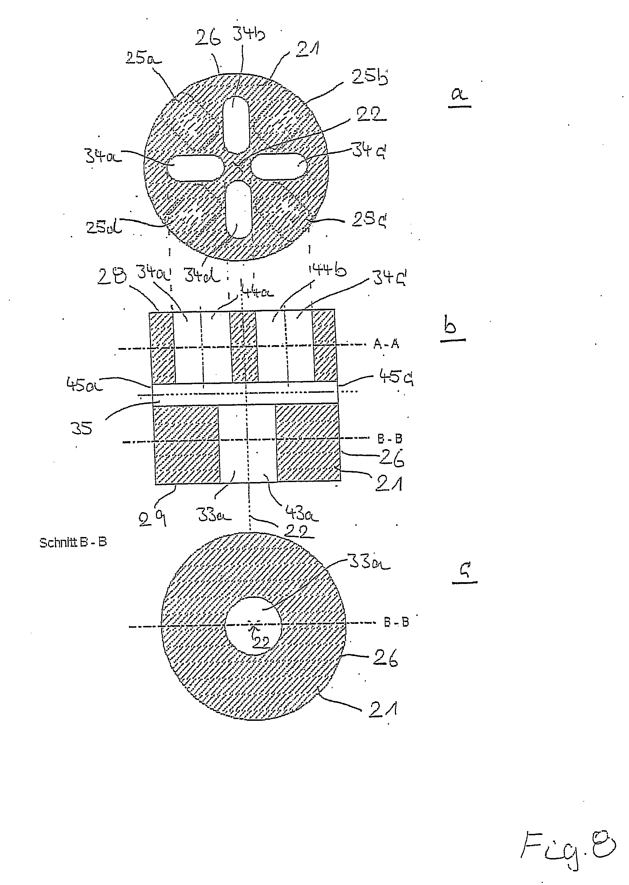

Fig. 8 zeigt ebenfalls ein Rührorgan, wie es ähnlich bereits in Fig. 3 dargestellt wurde. Fig. 8b zeigt dabei einen Querschnitt, Fig. 8a einen Querschnitt längs der Schnittlinie A-A in Fig. 8b und Fig. 8c einen Querschnitt längs der Schnittlinie B-B in Fig. 8b. Im Unterschied zu Fig. 3 liegen nunmehr jedoch nicht zwei vertikal von der Oberfläche des Rührorgans sich in dessen Innere erstreckende Bohrung 34a und 34b sondern insgesamt vier um einen Winkel von 90° zueinander versetzte Bohrungen 34a bis 34d vor.

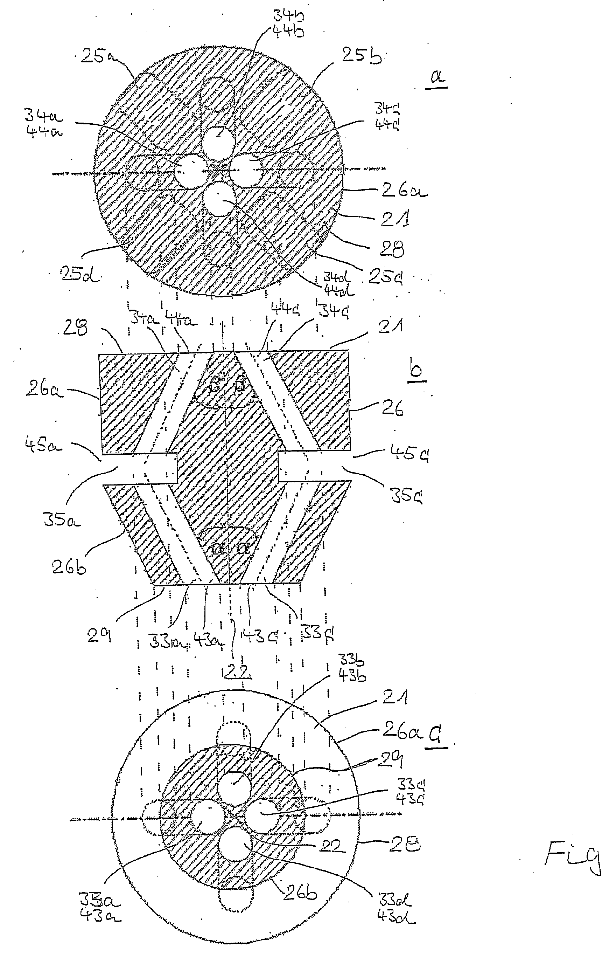

In Fig. 9 ist ein weiteres erfindungsgemäßes Rührorgan dargestellt, wobei die Figuren 9a und 9c eine Aufsicht bzw. eine Untersicht des in Fig. 9b im Querschnitt dargestellten Rührorgans darstellen. Auch hier sind in dem Rührer 21 wiederum vier von der Oberseite 28 des Rührorgans 21 ausgehende Bohrungen 34a bis 34d um die Mittelachse 22 des Rührorgane 21 um 90° versetzt angeordnet, wobei sich diese Bohrung 34a bis 34d nunmehr unter einem Winkel β zur Mittelachse bzw. Drehachse 22 des Rührorgans 21 nach unten außen erstrecken. In gleicher, nahezu symmetrischer Weise erstrecken sich Bohrungen 33a bis 33d von der Unterseite 29 des Rührorgans 21 unter einem Winkel α gegen die Mittelachse bzw. Drehachse 22 des Rührorgans 21 nach oben außen in den Körper des Rührorgans 21 hinein. Beide treffen auf insgesamt vier ebenfalls um 90° gegeneinander versetzt angeordnete horizontale Kanäle 35a bis 35d, die mit Öffnung 45a bis 45d in der Seitenfläche 26 des Rührorgans enden.

Die Seitenfläche 26 dieses Rührorgans erstreckt sich von seiner Oberfläche 28 senkrecht nach unten bis zu der Ebene der horizontalen Bohrungen 35a bis 35d und läuft dann konisch parallel zu den Bohrungen 33a bis 33d nach unten zusammen.

Die Bohrungen 33a bis 33d fördern wiederum Flüssigkeit vom Boden eines Gefäßes nach oben während die Bohrungen 34a bis 34d Gas- und Flüssigkeit bzw. eine Mischung hiervon von der Oberfläche 28 des Rührorgans 21 einziehen und an der Grenzfläche von Gas- und Flüssigkeit Turbulenzen erzeugen. Auch hierdurch wird ein pulsierender Bernoulli-Effekt erzeugt bzw. gegebenenfalls verstärkt.

Eine weitere Möglichkeit einen pulsierenden Bernoulli-Effekt zu erzeugen besteht darin, ein nicht rotationssymmetrisches Rührorgan einzusetzen.

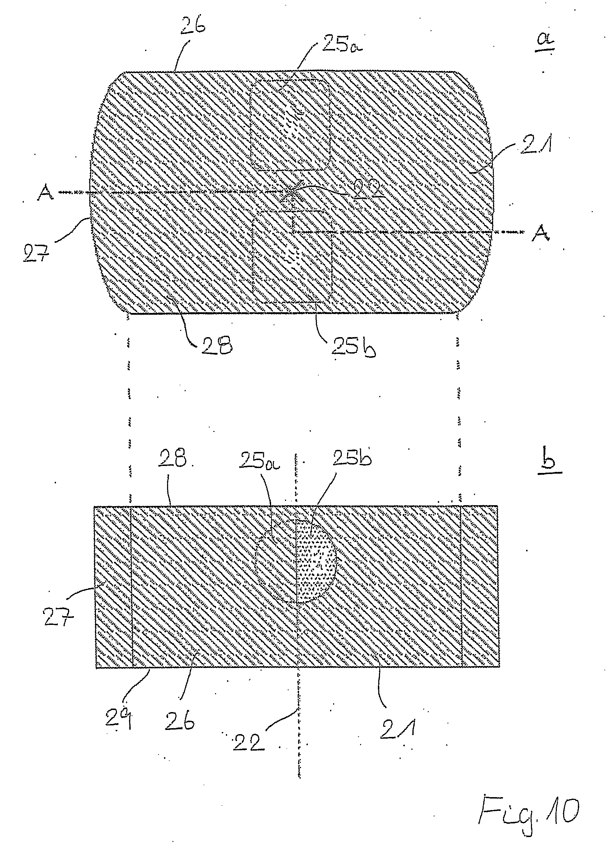

Bei dem in Fig. 10 dargestellten Rührorgan 21 liegen eine geradlinige Vorderseite 26 und eine zur Vorderseite 26 parallele, geradlinige Rückseite 27 vor, während die sich längs der Wand des Rührgefäßes 9 bewegenden die Vorderseite 26 und die Rückseiten 27 verbindenden Seitenflächen 27 konvex gekrümmt sind.

Ein derartiges Rührorgan 21 kann beispielsweise in einem nicht rotationssymmetrischen Rührgefäß oder in einem Rührgefäß mit Strömungsbrechern eingesetzt werden.

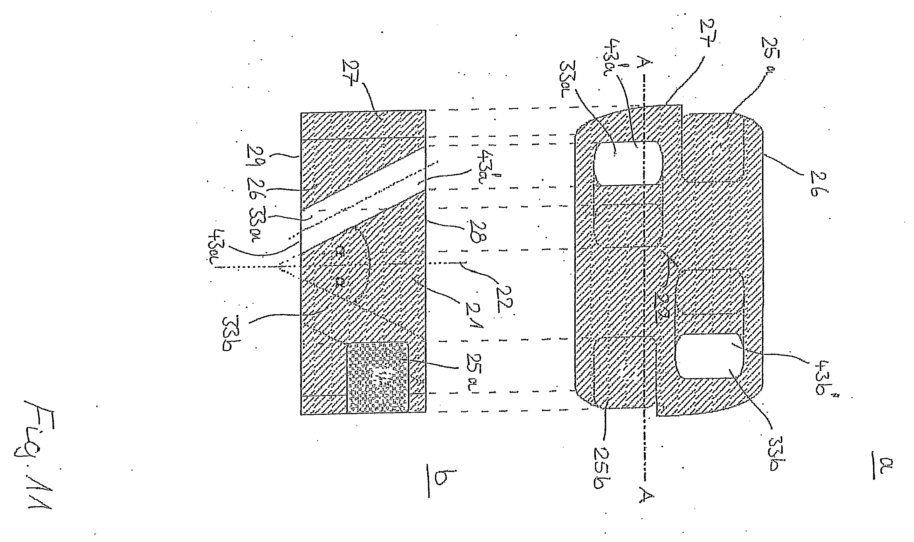

In Fig. 11 ist ein Rührorgan wie in Fig. 10 dargestellt, das zusätzlich unter einem Winkel α gegen die Drehachse 22 verlaufende Bohrungen 33a bzw. 33b aufweist. Diese fördern Flüssigkeit von den Öffnungen 43a, 43b an der Unterseite 29 des Rührorgans 21 zu den Öffnungen 43a' und 43b' an der Oberseite 28 des Rührorgans 21.

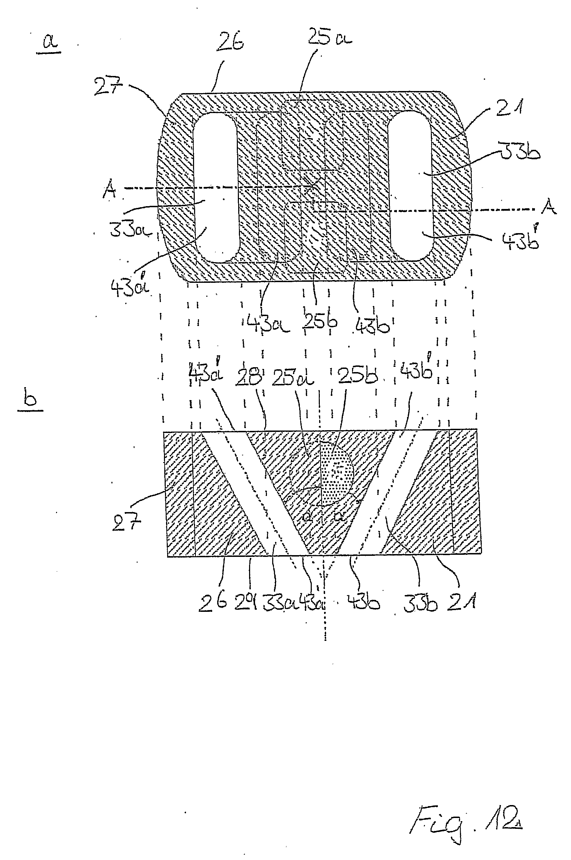

Fig. 12 zeigt ein Rührorgan wie in Fig. 11, wobei nunmehr die Bohrungen 33a und 33b eine wie in Fig. 12a in der Aufsicht erkenntliche im Querschnitt längliche Form aufweisen.

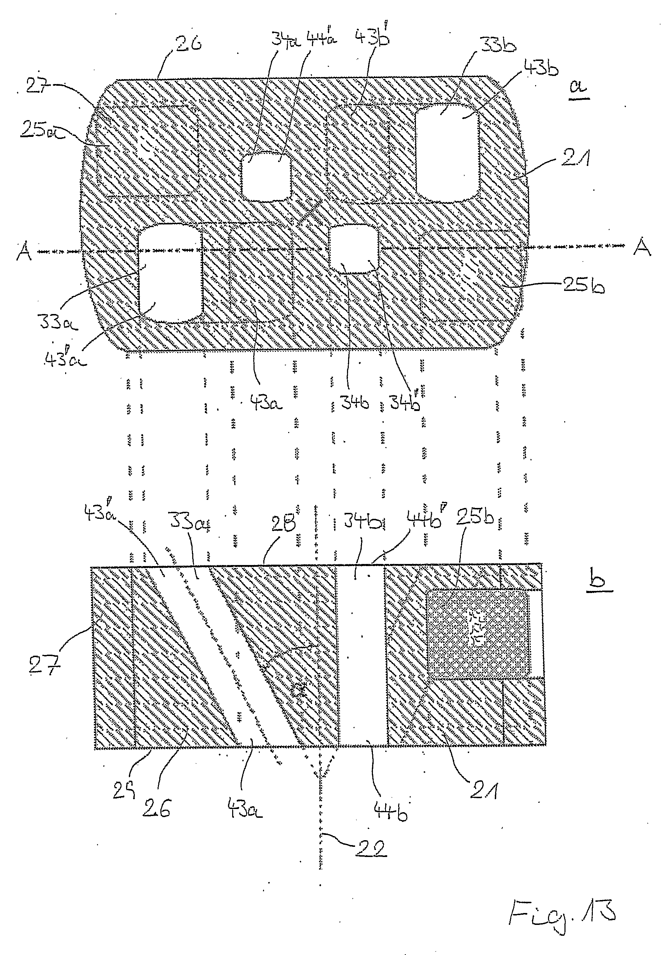

In Fig. 13 sind zusätzlich zu den Bohrungen 33a und 33b wie in Fig. 11 weitere Bohrungen 34a, 34b eingebracht, die sich versetzt zur Mittelachse 22 des Rührorgans 21 parallel zur Mittelachse 22 von der Unterseite 29 zur Oberseite 28 erstrecken und jeweils in Öffnungen 44a, 44b an der Unterseite 29 bzw. in Öffnungen 44a', 44b' an der Oberseite 28 enden. Diese Kanäle 34a, 34b dienen der Entgasung der Flüssigkeit, d. h. dem Entfernen der verbrauchten in die Flüssigkeit eindispergierten Gasblasen.

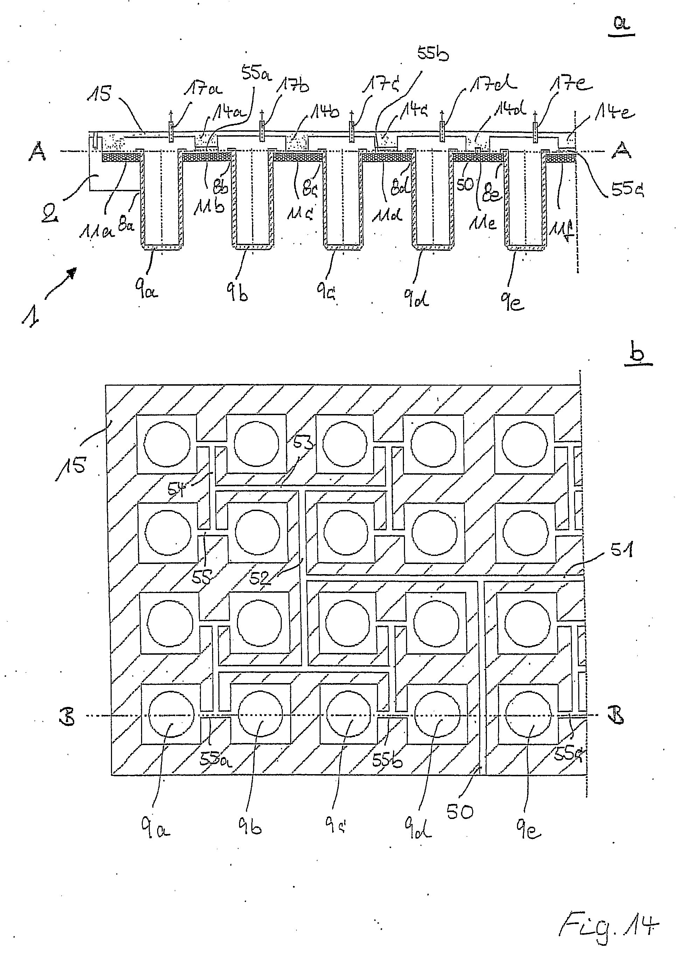

Fig. 14 zeigt in Teilbild a eine erfindungsgemäße Reaktorvorrichtung, bei der in Öffnungen 8a bis 8e in einem Reaktorblock 2 Behälter 9a bis 9e eingelassen sind. Diese Behälter 9a bis 9e weisen wiederum an ihrem oberen Umfangsrand ihrer Öffnung einen Flansch auf, mit dem sie auf einer Distanzscheibe 11a bis 11f aufliegen. Die gesamte Anordnung der Behälter wird überdeckt von einem deckelartigen Verschluss 15, in den wie bereits oben beschrieben, Röhrchen 17a bis 17e eingelassen sind, um ein Ausströmen von Gas zu ermöglichen. Der Deckel 15 weist weiterhin Trennwände 14a bis 14e auf, um die einzelnen Behälter voneinander gasdicht zu isolieren.

Fig. 14b zeigt nun einen Querschnitt längs der Linie A-A in Fig. 14a durch den Verschluss 15, während Fig. 14a ein Querschnitt durch die Gesamtanordnung längs der Linie B-B in Fig. 14b darstellt.

Ausgehend von einer Gaszufuhrbohrung 50 verästelt sich diese Bohrung über einzelne weitere Bohrungen 51, 52, 53, 54 bis 55 immer weiter und mündet letztlich in jeweils einen Gasauslass über den einzelnen Behältern 9a bis 9e. In Fig. 14b ist auch zu erkennen, dass die Behälter 9 in einem zweidimensionalen Array angeordnet sind. Die Länge der Bohrungen 50 bis 55 von dem Gaseinlass 50 bis zum Auslass in den Luftraum über den Gefäßen 9 ist dabei jeweils konstant. Auch die Zahl der Knicke, die die einzelnen Gasführungen vom Einlass bis zum Auslass über den Behältern durchlaufen, ist konstant. Die Höhe der Kanäle kann dabei jedoch variabel sein. Entscheidend ist nun, dass entweder in diesen Bohrungen 50 bis 55 kein nennenswerter Druckabfall auftritt oder der Druckabfall durch die gleiche Länge und die gleiche Anzahl an Knicke für jedes einzelne Behältnis 9 identisch ist.

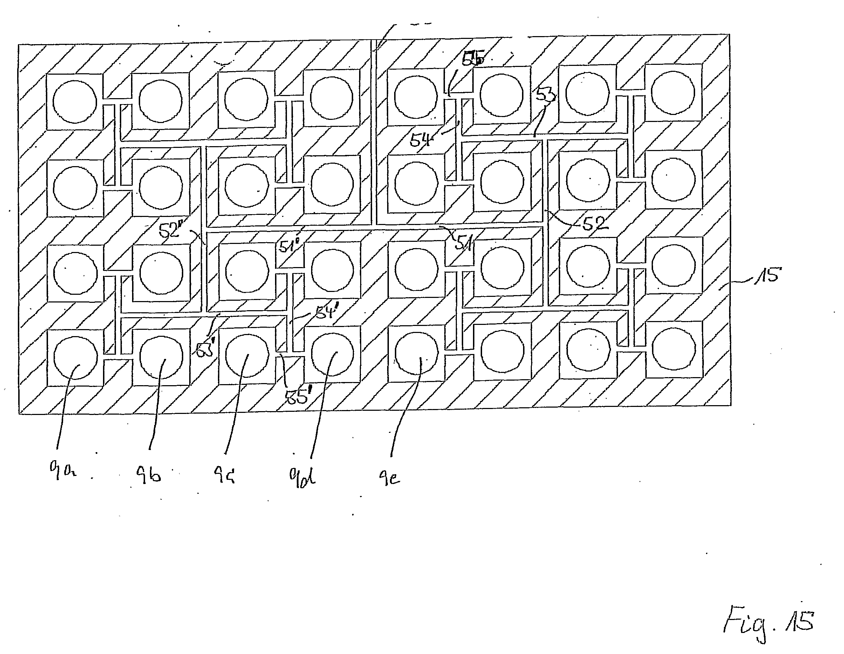

Fig. 15 zeigt einen weiteren Verschluss 15 mit einer entsprechenden Gasverteilerstruktur bestehend aus Kanälen 50 bis 55, 51' bis 55'. Auch hier sind die Wege und die Anzahl der Knicke vom Einlass 50 bis zu dem jeweiligen Behälter 9 für jeden Behälter identisch.

Im folgenden werden nun einzelne Messungen mit erfindungsgemäßen Reaktionsgefäßen bzw. mit einem erfindungsgemäßen Bioreaktorblock dargestellt.

Wenn der Bioreaktorblock 1 entsprechend geometrisch gestaltet wird (Abstand und Anordnung der einzelnen Milliliter-Rührreaktoren, z. B. jeweils 8 Milliliter-Rührreaktoren parallel - da Pipettier-Roboter meist mit 8 parallelen Dosierstrecken ausgerüstet sind -, etc.) kann eine einfache Automatisierung erfolgen. Mit Hilfe eines Pipettier-Roboters als Aktor können
- Proben individuell gezogen und prinzipiell alle manuellen off-line Analysenverfahren automatisiert werden (Bestimmung von Zell-, Substrat-, Produkt- und Nebenprodukt-Konzentrationen),
- Korrekturmittel, Substrate und/oder Induktoren individuell dosiert werden,
- die einzelnen Milliliter-Rührreaktoren mit Sensoren (bspw. entsprechend dimensionierte pH-Elektroden) intermittierend beprobt werden.

Mit Hilfe geeigneter Prozesskontrollsysteme ist damit eine automatisierte Prozessführung im Parallelansatz möglich: pH-Regelung, individuelle Substratdosierung, automatisierte off-line Probenahme und Analyse, und dergleichen.

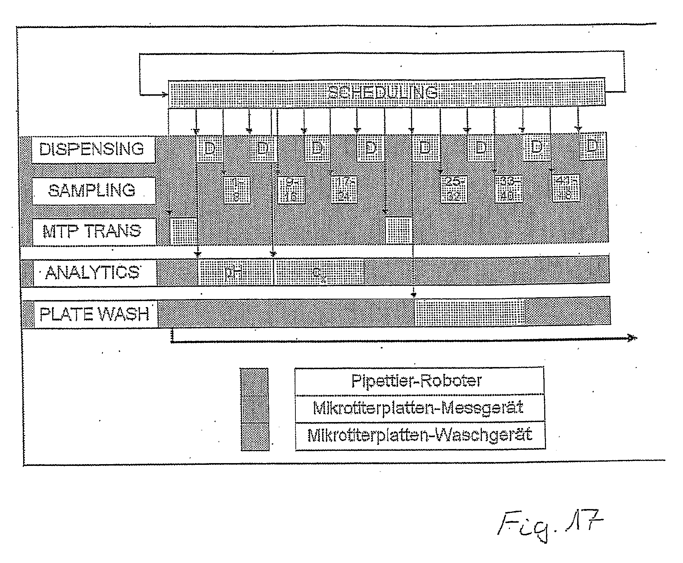

Prinzipiell sind zur automatisierten Durchführung von parallelen Zellkultivierungen folgende Arbeitsschritte notwendig. Die Zeitplanung sind dabei so ausgelegt, dass die Arbeitsschritte im Verlauf des gesamten Prozesses im gleichen Zeittakt ausgeführt werden können, d.h. es wird die Einhaltung einer konstanten Zeitspanne Δt zwischen den Arbeitsschritten ermöglicht. Ein solcher Zeitplan ist in Fig. 17 dargestellt.
- Dispensing D: Die parallel betriebenen Milliliter-Rührreaktoren werden von einem Pipettier-Roboter mit sterilem, destillierten Wasser zur Verdunstungskontrolle und/oder frischem Medium zur Realisierung von Zulauf- oder kontinuierlichen Verfahren und/oder Titrationsmittel zur pH-Kontrolle und/oder Induktionsmittel versorgt. Die jeweilig notwendigen Volumina einer jeden Lösung werden durch im Voraus mit allen notwendigen Parametern initialisierte Algorithmen berechnet.
- Sampling: Um at-line Analytik automatisiert durchführen zu können, werden den Milliliter-Rührreaktoren regelmäßig vorgegebene Probenvolumina entnommen und in Mikrotiterplatten abgegeben.
- Mikrotiterplattenbewegungen MTP Trans: Nach Entnahme einer vorgegebenen Anzahl Proben in eine Mikrotiterplatte, beispielsweise einer Probe pro Milliliter-Rührreaktor, wird die Mikrotiterplatte vom Labor-Roboter zu einem Mikrotiterplatten-Messgerät transportiert.
- At-line Analytik: Die in eine Mikrotiterplatte abgegebenen Bioreaktorproben werden in einem Messgerät (z.B. Mikrotiterplatten-Photometer/Fluorimeter) vermessen. Beispielsweise können so die Biotrockenmassekonzentration (cₓ) und der pH-Wert bestimmt werden. Auch andere bioverfahrenstechnisch relevante Parameter (z.B. Substratkonzentration, Produktkonzentration) könnten auf diese Weise bestimmt werden. Die erhaltenen Messwerte werden Regelalgorithmen zur Verfügung gestellt und nehmen so Einfluss auf die folgenden vom Laborroboter auszuführenden Dispensing-Schritte. Durch Etablierung eines Kreislaufs mindestens zweier Mikrotiterplatten kann die Analytik parallel zu den vom Pipettier-Roboter durchgeführten Dispensing- und Sampling-Schritten erfolgen. Eine Mikrotiterplatte wird demnach mit Proben gefüllt während die Proben in der zweiten Mikrotiterplatte im Messgerät analysiert werden. Nach abgeschlossener Analyse der Mikrotiterplatte wird diese in einem Mikrotiterplatten-Waschgerät gesäubert und dem Kreislauf erneut zugeführt.
- Reinigung der Mikrotiterplatte ("Plate wash"): Nach Beendigung des Versuchs wird die jeweilige Mikrotiterplatte gereinigt und kann so erneut verwendet werden.

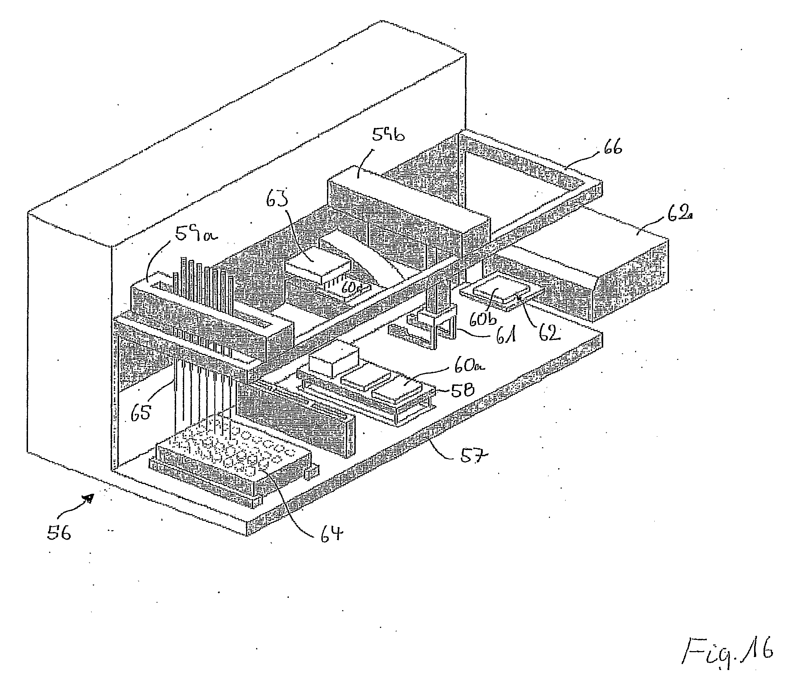

Fig. 16 zeigt eine Anordnung mit Mikrotiterplatten 60a, 60b, 60c in einem Pipettier-Roboter, wobei in dieser Skizze der Kreislauf einer Proben-Mikrotiterplatte 60a bis 60c verfolgt werden kann. Die in Fig. 16 dargestellte automatische Anlage 56 weist eine Grundplatte 57 auf, auf der eine Anordnung 64 von Behältern (Reaktorblock) oder Probenahmegefäßen, ein Tisch 58 zur Aufnahme von Mikrotiterplatten 60a, ein Fotometer 62 und eine Waschstation 63 aufweist. Weiterhin ist oberhalb der Grundplatte 57 ein Tragebalken 66 angeordnet, auf dem mittels Querbalken 59a und 59b die Pipettenspitzen 65 und ein Tragarm 61 für Mikrotiterplatten verschieblich aufgehängt sind.

In einem ersten Schritt wird nun eine Mikrotiterplatte 60a mit Proben aus dem Reaktorblock 64 über die Pipettenspitzen 65 befüllt. Die Mikrotiterplatte 60a wird dann mit dem Tragarm 61 zu dem Fotometer 62 transportiert, wo die einzelnen Näpfchen der Mikrotiterplatten 60b fotometrisch vermessen werden. Die so vermessene Mikrotiterplatte 60b wird mit dem Tragarm zu der Waschvorrichtung 63 transportiert, wo die Mikrotiterplatte (hier nun Mikrotiterplatte 60c) gewaschen und gereinigt wird. Damit steht die Mikrotiterplatte 60c wiederum den Proben und Messzyklus zur Verfügung und wird von dem Tragarm 61 zurück auf den Tisch 58 befördert.

Je nach Prozessanforderung können dem Prozesskreislauf zu bestimmten Zeitpunkten mit Proben gefüllte Mikrotiterplatten entzogen und gekühlt zwischengelagert werden. Eine neue Mikrotiterplatte kann dem Prozess automatisch zugeführt werden, um den Analytikkreislauf aufrecht erhalten zu können.

Die Bestimmung von im wässrigen Reaktionsmedium gelösten Stoffen wie die Wasserstoffionenaktivität (pH) muss in jedem Reaktionsgefäß individuell erfolgen. Eine Verwendung von 48 oder 96 einzelnen pH-Sensoren, beispielsweise sterilisierbare pH-Einstabmessketten (Glaselektroden), ist nicht ökonomisch. Auch die Verwendung von kostengünstigen pH-Feldeffekttransistoren ("Wegwerfsensoren") ist aufgrund der zusätzlich erforderlichen klassischen Referenzelektroden und der fehlenden thermischen Stabilität (Sterilisierbarkeit) praktisch nicht möglich.

Die Anzahl der erforderlichen pH-Sensoren für Parallelreaktoren kann prinzipiell reduziert werden, wenn ein Sensor für mehrere Reaktionsgefäße benutzt werden kann. Eine Möglichkeit der technischen Realisierung ist die Integration kommerziell erhältlicher Miniatur-pH-Elektroden in Anstechkanülen, die mit Hilfe eines Pipettierroboters intermittierend in die einzelnen Milliliter-Rührreaktoren eingetaucht werden. Hierzu geeignet sind pH-Einstabmessketten mit einem Außendurchmesser von 1 mm und einer Ansprechzeit von ∼ 6 s.

Eine weitere Möglichkeit ist die sterile Entnahme von Proben aus den einzelnen Milliliter-Rührreaktoren mit Kanülen und die parallele Messung des pH-Wertes in den Proben mit pH-sensitiven Mikrotiterplatten. Am Boden der Kavitäten dieser kommerziell erhältlichen Mikrotiterplatten ist ein Sensorspot integriert, in welchem zwei Fluorophore immobilisiert sind. Diese Fluorophore können in einem entsprechend ausgestatteten Photometer-Fluorimeter ausgelesen werden.
Die Fluoreszenzeigenschaften des Indikator-Fluorophors variieren mit dem pH-Wert der Lösung während der Referenz-Fluorophor ein vom pH-Wert unabhängiges Fluoreszenzsignal produziert. Das Verhältnis aus Indikator- zu Referenzsignal kann zum pH-Wert der Lösung über eine Sigmoid-Funktion korreliert werden. Die Verwendung eines Referenzfarbstoffs erhöht die Messgenauigkeit und die Lebensdauer der Sensoren, da eine Abnahme der Signalintensität durch "Ausbluten" des Sensors (Diffusion der Fluorophore in die Messlösungen) einen geringeren Einfluss auf das Messsignal zur Folge hat.

Die erzeugten pH-Messdaten werden vom Prozesskontrollsystem gelesen und Regelalgorithmen zur Verfügung gestellt. Diese berechnen das notwendige Volumen Titrationsmittel pro Reaktorgefäß um einen gewünschten pH-Sollwert in dem Reaktorgefäß aufrecht zu erhalten. Das Prozesskontrollsystem berechnet die Dispensing-Schritte unter Berücksichtigung der notwendigen Zudosierung zur pH-Kontrolle.

Wesentlich an der vorliegenden Erfindung ist der damit mögliche hohe Sauerstoff- und Energieeintrag in eine Kultursuspension. Im folgenden werden daher Messungen der Sauerstoffübergangskoeffizienten k_{L}a verschiedener erfindungsgemäßen Magnetrührsysteme beschrieben.

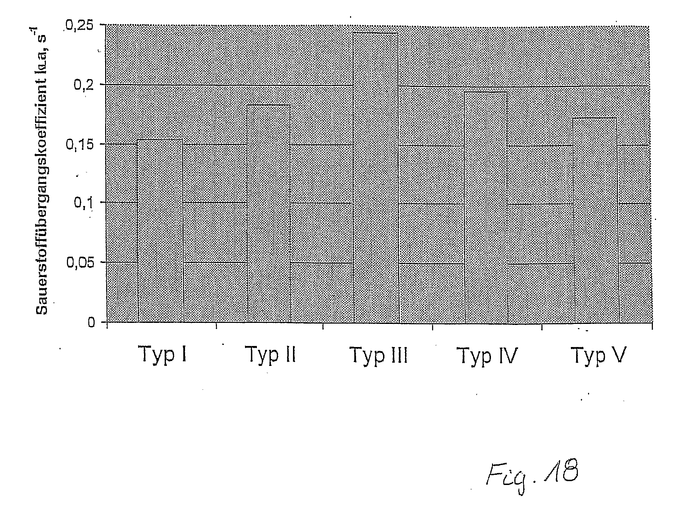

Es wurden Rührorgane der Typen I bis V entsprechend Fig. 7 (Typ I), Fig. 10 (Typ II), Fig. 12 (Typ III), Fig. 11 (Typ IV) und Fig. 9 (Typ V) mit der dynamischen Sulfitmethode (Havelka et al. 1998) unter vergleichbaren Bedingungen bzgl. Volumen der Flüssigphase, Lagerung des Rührorgans und Anordnung der Strömungsbrecher verglichen (siehe Fig. 18). Diese Versuche wurden in einem Milliliter-Rührreaktor mit 15,5 mm Durchmesser durchgeführt. Mit den Rührern der Typen I bis V wurden bei einer Drehzahl von 2600 rpm k_{L}a-Werte in 0,5 M Na₂SO₄-Lösung im Bereich von 0,156-0,244 s⁻¹ erreicht.

Zur Bestimmung der k_{L}a-Werte wird als Flüssigphase 0,5 M Na₂SO₄-Lösung verwendet, weiche nicht-koaleszierende Bedingungen garantiert. Zusätzlich wird eine Konzentration von 10⁻³ M CoS04 als Katalysator für die chemische Oxidation von Sulfit zu Sulfat vorgelegt. Die Durchführung der dynamischen Sulfitmethode beginnt mit dem Belüften der Flüssigphase mit Luft, bis diese Sättigung erreicht. Nach Zugabe einer ausreichend großen Stoffmenge Sulfit um den gesamten, in der Flüssigphase gelösten Sauerstoff zu verbrauchen, sinkt die Gelöstsauerstoffkonzentration der Flüssigphase schlagartig auf Null. Nach stöchiometrischem Sulfitumsatz steigt die Gelöstsauerstoffkonzentration in der Flüssigphase wieder an. Aus dieser sogenannten Aufsättigungskurve wird der k_{L}a-Wert unter Annahme ideal durchmischter Bedingungen in Flüssig- und Gasphase bestimmt. Dabei wird die Ansprechzeit der verwendeten Sauerstoffsonde im Modell berücksichtigt.

Herkömmliche technische Rührkesselreaktoren werden bei Sauerstoffübergangskoeffizienten von k_{L}a < 0,25 s⁻¹ betrieben. Damit sind in erfindungsgemäßen Milliliter-Rührreaktoren bzw. mit den erfindungsgemäßen Rührorganen dieselben oder ähnliche Sauerstoffeintragsraten erreichbar (zum Vergleich: in Schüttelkolben oder Mikrotiterplatten können unter optimalen Bedingungen Sauerstoffübergangskoeffizienten von maximal k_{L}a = 0.07.s⁻¹ erreicht werden).

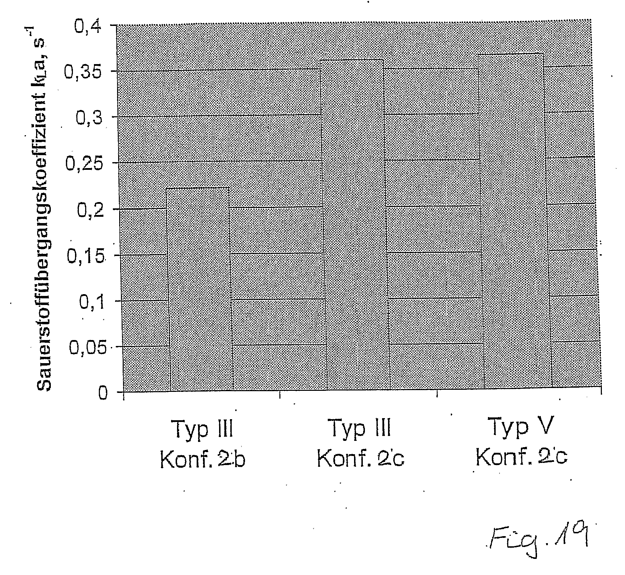

Sauerstoffübergangskoeffizienten der Rührsysteme des Typs III und V wurden darüber hinaus mit 8 ml 0,5 M Na₂SO₄-Lösung ebenfalls in einem Milliliter-Rührreaktor mit 20 mm Durchmesser durchgeführt (Fig. 19). Dabei wurden Rührsysteme nach Fig. 2b und 2c verwendet. Maximal erreichte k_{L}a-Werte für eine Drehzahl von 2800 rpm liegen hier sogar bei bis zu 0,355 s⁻¹.

Zur Verifizierung der Sauerstoffeintragseigenschaften der Rührsysteme wurden parallele Kultivierungen mit Escherichia coli (wt) als Beispielsystem durchgeführt.

100 ml eines sterilen, definierten Mediums wurden in einem mit Alucap verschlossenen 500 ml Schüttelkolben mit 0,25% Inokkulum aus dem Feedstock angeimpft und 14 h bei 37 °C und 200 rpm in einem Schüttelinkubator mit einer Exzentrizität von 5 cm inkubiert. Am folgenden Tag wurden 4-6 ml dieser Vorkultur in Milliliter-Rührreaktoren überführt. In den Milliliter-Rührreaktoren wurden die Zellen für 3,5 h bei 2000 rpm eines erfindungsgemäßen Rührorgans und weitere 2,5 h bei 2200 rpm bei 37 °C inkubiert. Der pH-Wert wurde auf 6,8 geregelt.

Der Schüttelkolben (SK) wurde mit den ca. 40 ml Restvolumen der Vorkultur weiter im Schüttelinkubator bei gleichen Bedingungen inkubiert.

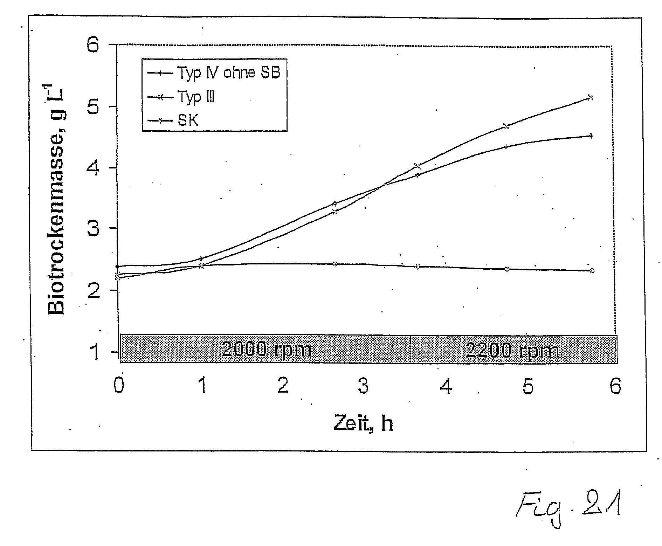

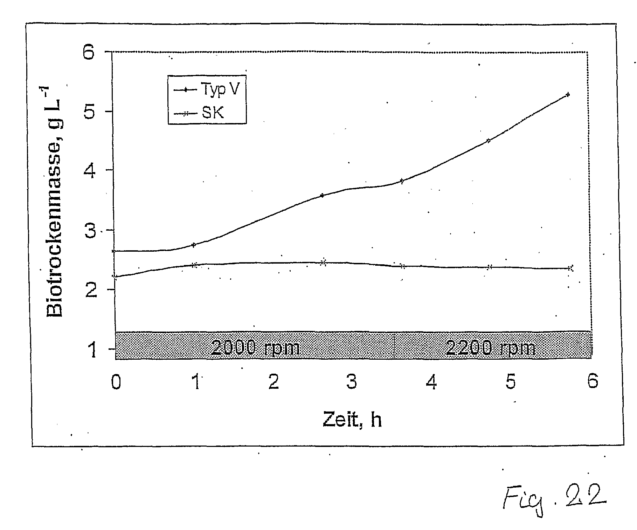

Die Messergebnisse in Fig. 20 bis 22 belegen, dass in den Milliliter-Rührreaktoren ein weiteres Wachstum der Bakterien möglich ist, wenn genügend Sauerstoff eingetragen wird. Dabei zeigen die jeweiligen Kurven einen herkömmlichen Schüttelkolben (SK), einen erfindungsgemäßen Rührer eines bestimmten Typs ohne Strömungsbrecher (z. B. "Typ II ohne SB") bzw. mit Strömungsbrecher (z. B. Typ II"). Aus den Fign. 20 bis 22 ist zu erkennen, dass bereits eine Drehzahl von 2200 rpm ermöglichte in den Ansätzen mit den Rührsystemen des Typs II, III und V eine bis zu 2,5-fach höhere Biotrockenmassekonzentration, als im Schüttelkolbenansatz (SK) ermöglicht.

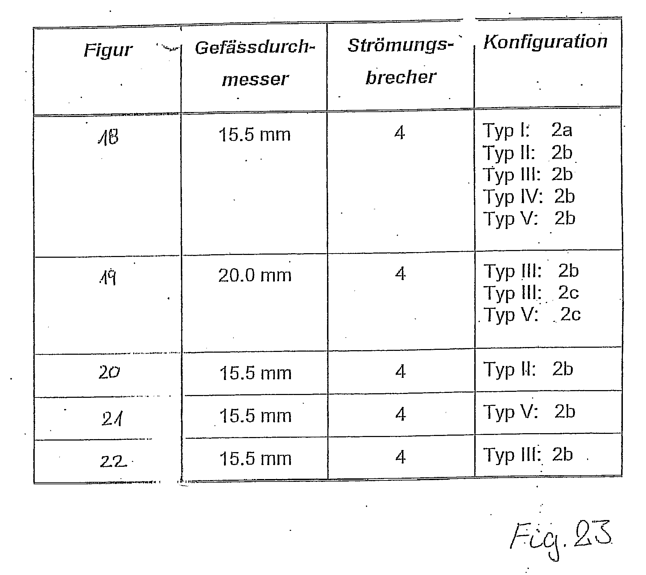

Fig. 23 gibt in Tabellenform nun jeweils an, in welcher Konfiguration die einzelnen Rührsysteme eingesetzt wurden. Dabei wird jeweils für die Messungen, die in einer bestimmten Figur dargestellt sind, der Gefäßdurchmesser und die Anzahl der Strömungsbrecher angegeben. Weiterhin wird die Konfiguration aus Strömungsbrecher und Rührorgan unter Bezug auf die Darstellung in den Fign. 2a bis 2d angegeben.

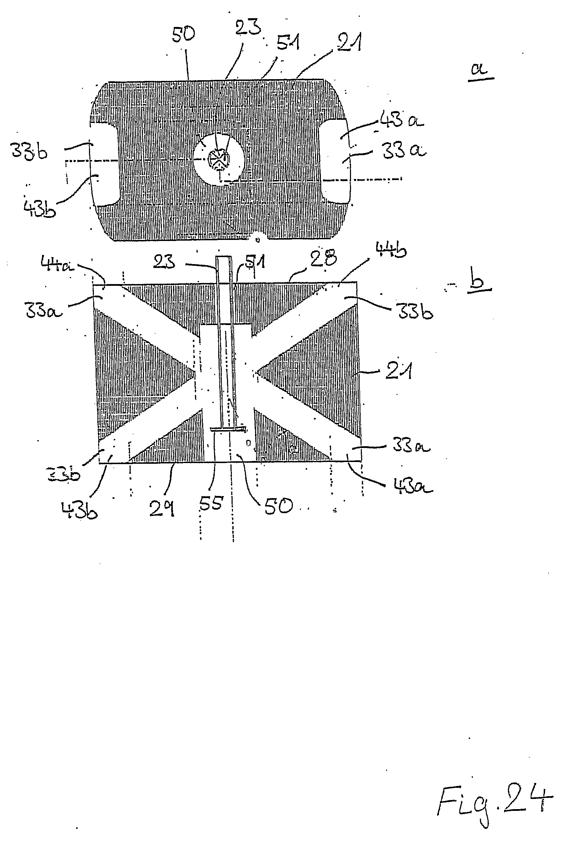

Die Figuren 24 bis 26 zeigen weiteren Ausgestaltungen erfindungsgemäßer Rührorgane. Diese Rührorgane sind sämtlich zur Lagerung auf einer Welle 23 vorbereitet, indem Bohrungen 50 und 51 in das Rührorgan 21 eingebracht sind. Im oberen Bereich des Rührorgans besitzt die Bohrung 51 eine lichte Weite, so dass sie den Durchtritt und die Halterung der Welle 23 ermöglicht. Figur 24a zeigt nun eine Aufsicht, wobei die unterschiedlichen Durchmesser der Bohrungen 50 und 51 eingezeichnet sind. Figur 24b zeigt eine Querschnittsansicht desselben Rührorgans.

Die Bohrungen 33a und 33b sind nunmehr von einer Unterkante des Rührorgans zur gegenüberliegenden oberen Kante des Rührorgans durchgezogen. Die Bohrungen 33a und 33b liegen dabei in einer Schnittebene, so dass sie sich im Bereich der Bohrung 50 überkreuzen und einen gemeinsamen Hohlraum bilden. Die Öffnungen der Bohrungen 33a und 33b erstrecken sich nunmehr sowohl über einen Teil der Unterseite 29 bzw. der Oberseite 28 des Rührorgans 21 als auch über seine Seitenwand. Im Ergebnis laufen also vier Teilbohrungen sternförmig von gegenüberliegenden Seiten an der oberen und der unteren Kante des Rührorgans in der Mitte aufeinander zu.

Dieses Rührorgan kann selbstzentrierend oder auch wie in Figur 24 dargestellt an einer in das Reaktionsgefäß eingebrachten Achse 23 gelagert werden. Die Achse 23 kann dabei beispielsweise am Deckel des Reaktionsgefäßes befestigt werden. In Figur 24 weist die Achse 23 an ihrem in der Bohrung 50 befindlichen unteren Ende eine Verdickung bzw. einen Flansch 50 auf, so dass das Rührorgan nicht von der Achse 23 fallen kann, da sich die Bohrung 50 im oberen Bereich übergehend in die Bohrung 51 verengt. Diese Verdickung 55 kann ebenfalls als Schlüsselfläche ausgeführt sein. Die Achse, die nunmehr abweichend von den bisherigen Ausführungsformen von oben in das Reaktionsgefäß hineinragt und in die Flüssigphase eintaucht, kann wie in Figur 24 als Vollmaterial ausgeführt sein. Wesentlich ist dabei eine exakte Positionierung des Achsenendes relativ zur Innengeometrie des Rührsystems, um eine effektive Sogwirkung zu erzielen und damit den Blaseneintrag zu intensivieren.

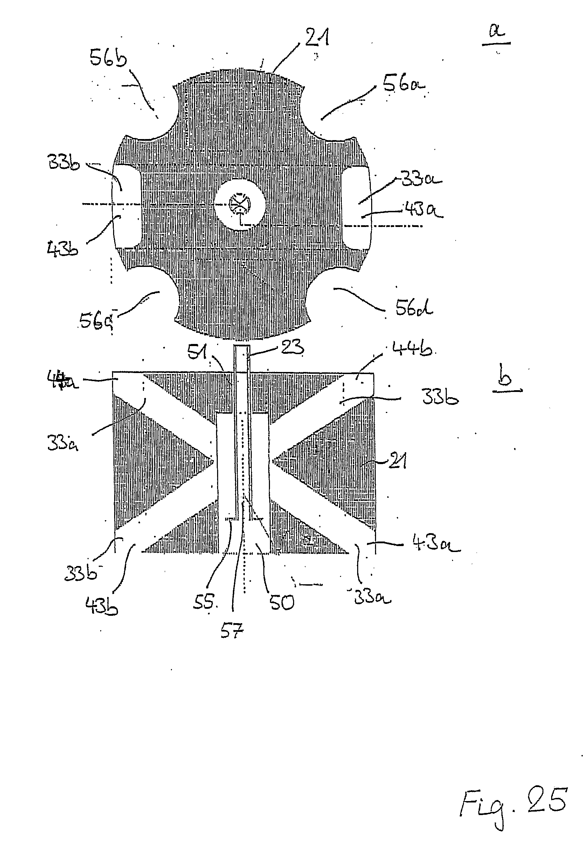

Figur 25 zeigt ein ähnliches Rührorgan wie in Figur 24 mit dem Unterschied, dass die Außenform des Rührorgans, wie in dem Querschnitt in Figur 25a zu erkennen, prinzipiell kreisförmig ist. Dieses Rührorgan weist nunmehr zusätzlich Ausnehmungen 56a bis 56b auf, die beim Umlauf in der Strömung eine zeitliche bzw. örtliche Änderung der Strömungsgeschwindigkeit längs des Umfangs des Rührorgans 21 bewirken. Dadurch wird eine noch bessere Durchmischung und Blaseintrag erzielt. Weiterhin ist, wie in dem seitlichen Querschnitt aus Figur 25b zu erkennen, die Achse 23 als Hohlrohr ausgeführt, so dass über den zentralen Hohlraum 57 ein Gastransport von oben nach unten in die Flüssigphase stattfindet.

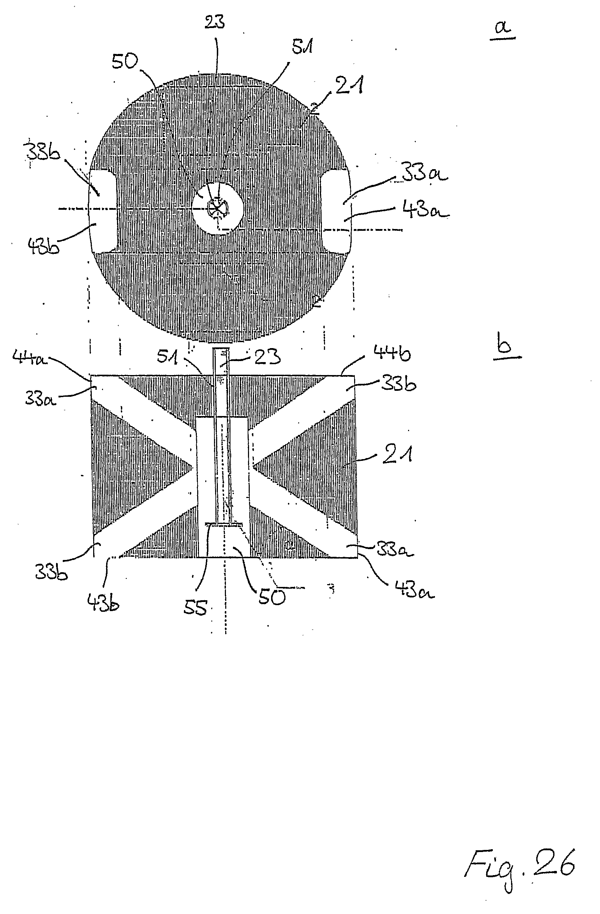

Figur 26 zeigt ein entsprechendes Rührorgan wie in Figur 25, wobei jedoch keine Ausnehmungen 56a bis 56b vorgesehen sind und die Achse 23 aus Vollmaterial besteht wie in Figur 24.

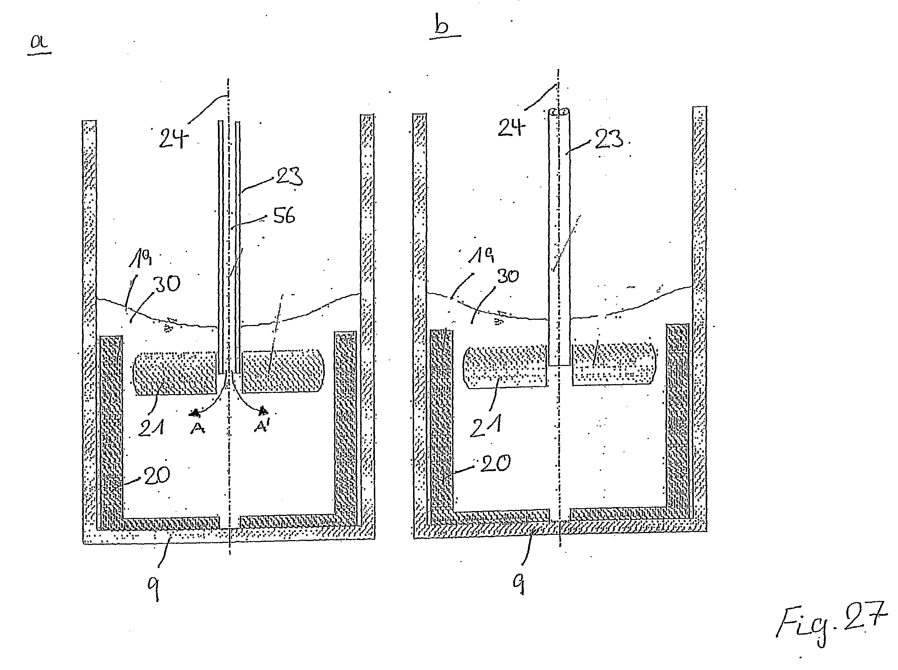

Figur 27 zeigt nun in den Teilfiguren a) und b) die beiden Varianten der Befestigung der Rührorgane mit von oben in die Flüssigphase 30 eintauchenden Achsen 23. Die Achse 23 in Figur 27a ist als Hohlrohr ausgebildet, so dass entsprechend den Pfeilen A und A' ein Gaseintrag in die Flüssigphase stattfindet. In Figur 27b ist die Achse 23 als Vollmaterial ausgebildet, so dass sie lediglich der Halterung und ggf. dem Drehen des Rührorgans 21 dient.

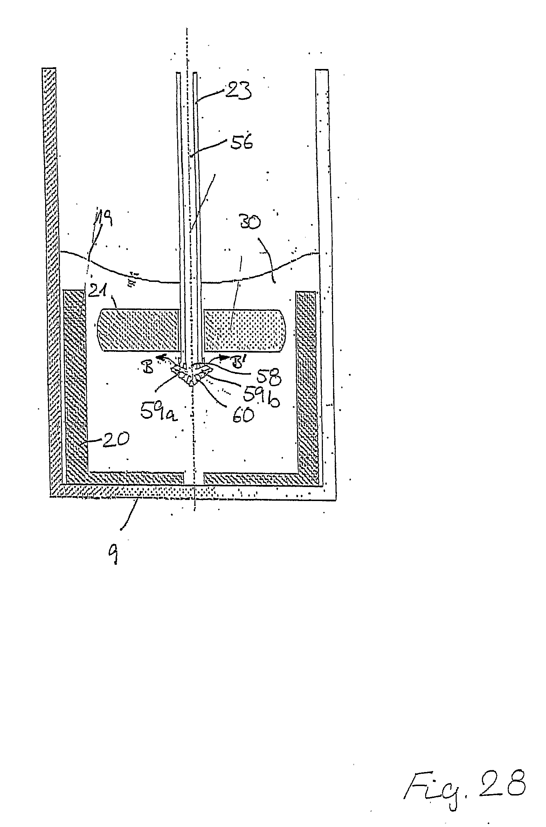

Figur 28 zeigt eine Vorrichtung gemäß Figur 27a, wobei jedoch zur weiteren Intensivierung des Sauerstoffeintrags und der Blasenbildung in der Flüssigphase 30 am unteren Ende der Achse 23 eine Düse 60 angeordnet ist. Diese kann entweder, wie in Figur 28 gezeigt, starr auf der Welle 23 angeordnet sein oder ggf. auch gleichzeitig als Anschlag (Flansch) für das Rührorgan 21 dienen. Sie kann weiterhin auch in das rotierende Rührorgan 21 eingepresst werden, statt an der Achse 23 angeordnet zu sein. Die Düse 60 enthält eine Luftübernahmebohrung 58, die den Hohlraum 56 der Welle 23 mit dem Kopf des Kegels der Düse 60 verbindet. Aus diesem Kopf des Düsenkegels 60 führen mehrere Austrittsbohrungen 59a und 59b aufwärts bzw. in Richtung des Rührorgans 21, wobei Luft in Richtung der Pfeile B und B' in die Flüssigphase 30 eingetragen wird. Auch hier ist eine exakte Ausrichtung relativ zum Rührsystem für einen hohen Sauerstoffübergang wichtig.

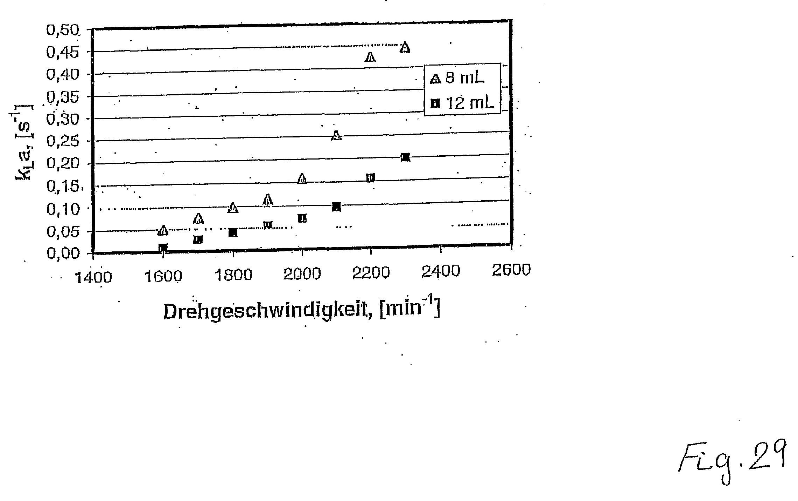

Figur 29 zeigt den maximalen Sauerstoffübergangskoeffizienten, der mit einem Rührsystem gemäß Figur 25 bei 8 bzw. 12 ml Reaktionsvolumen erreicht werden kann. Insbesondere bei einem Reaktionsvolumen von lediglich 8 ml kann bereits bei einer Drehzahl von 2300 Upm ein Sauerstoff ein Sauerstoffübergangskoeffizient von 0,4 s⁻¹ erzielt werden.

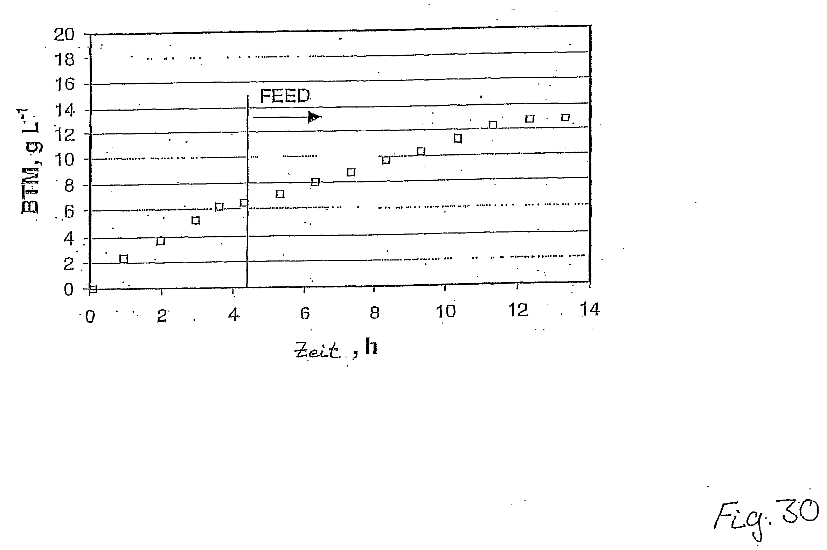

Figur 30 zeigt das Wachstum der Biomassekonzentration BTM in einer Fed-batch Kultivierung mit Escherichia coli wt K12. Mit Zufütterung wurde innerhalb von 9 Stunden eine Biotrockenmassenkonzentration von über 13 gL⁻¹ erreicht. Die Drehzahlen des Rührsystems, das demjenigen in Figur 25 entsprach, lagen dabei nicht über 2200 Upm, was dennoch einen Sauerstoffgehalt des Mediums von über 20% Luftsättigung sicherstellte.

Die Kultivierung erfolgte hier in einem mineralischen Medium mit 15 gL⁻¹ Glukose zu Beginn der Satzphase als Ausgangskonzentration. Ab Verbrauch dieser Glukose nach 4,15 h wurde intermittierend alle 4 Minuten eine Glukoselösung mit 250 gL⁻¹ Glukosegehalt zugefüttert. Der pH-Wert wurde mittels 2,5%iger NH_{4OH} auf 6,8 eingestellt.

Zusammenfassend lässt sich damit feststellen, dass durch die vorliegende Erfindung parallele Milliliter-Rührreaktoren ermöglicht wurden, in denen ein vergleichbarer Wachstumsverlauf von kontrollierten Prozessverläufen ermöglicht wird wie in Bioreaktoren das Labormaßstabs.

Oberflächenbegaste, gegebenenfalls mit speziellen Magnetrührorganen ausgestattete Milliliter-Rührreaktoren erlauben eine ebenso effiziente Sauerstoffversorgung von Organismen in Flüssigkultur wie Rührkesselreaktoren größeren Maßstabs mit Volumenbegasung.

Der Einsatz von bis zu 96 und mehr Milliliter-Rührreaktoren in einem Bioreaktorblock, der mit Hilfe von Pipettier-Robotern automatisierbar ist, ermöglicht erstmalig einen effizienten, individuell kontrollierten Parallelbetrieb. Die Verwendung eines LaborRoboters zur Automatisierung der Parallelreaktionen ermöglicht damit einen Quantensprung in der Gewinnung relevanter Prozessdaten.

Durch Implementierung geeigneter Screening- und Optimierroutinen können Screening-Verfahren und Prozessoptimierungen (Medienzusammensetzung, Induktionsverfahren, Dosierprofile) systematisch und mit hoher Zeiteffizienz im Parallelansatz automatisiert werden. Eine vollständige Digitalisierung der parallelen Prozessentwicklung ermöglicht weiter eine neuartige Datentransparenz und -verfügbarkeit.

Mit Hilfe dieses neuen Werkzeuges zur Hochdurchsatz-Bioprozessentwicklung können neue Bioprozesse zeiteffektiv unter technischen Reaktionsbedingungen entwickelt werden, da beispielsweise statt einem Experiment im kontrollierten 0,5 l Rührkesselreaktor mit demselben Reaktionsvolumen zeitgleich 100 Experimente in 100 parallelen 5 ml-Rührkesselreaktoren automatisiert durchführbar sind - also der 100-fache Informationsgewinn pro Zeiteinheit möglich wird. Bei Einsatz geeigneter Versuchsplanungsalgorithmen wird die Effektivität der Bioprozessentwicklung weiter gesteigert.

## Patentansprüche

1. Rührorgan mit einem Grundkörper, der im Betrieb eine Oberseite und eine Unterseite aufweist
**dadurch gekennzeichnet, dass**
in dem Grundkörper mindestens ein erster Durchgangskanal angeordnet ist, dessen erste Öffnung sich zumindest teilweise an der Unterseite des Grundkörpers und dessen zweite Öffnung sich an der Oberseite und/oder seitlich am Grundkörper befindet, wobei das Rührorgan derart ausgebildet ist, dass die Strömungsgeschwindigkeit sich längs einer Stromlinie oder Bahnlinie um die Rührachse örtlich und/oder zeitlich ändert.

2. Rührorgan nach dem vorhergehenden Anspruch, **dadurch gekennzeichnet, dass** in dem Grundkörper als erster Durchgangskanal mindestens eine erste Bohrung angeordnet ist, deren Durchgangsachse mit der Drehachse des Rührorgans einen zur Oberseite des Rührorgans sich öffnenden Winkel α mit 0° ≤ α < 90° einschließt.

3. Rührorgan nach einem der Ansprüche 1 oder 2, **dadurch gekennzeichnet, dass** in dem Grundkörper mindestens ein weiterer zweiter Durchgangskanal angeordnet ist, dessen erste Öffnung sich zumindest teilweise an der Oberseite des Grundkörpers und dessen zweite Öffnung sich an der Unterseite und/oder seitlich am Grundkörper befindet.

4. Rührorgan nach dem vorhergehenden Anspruch, **dadurch gekennzeichnet, dass** in dem Grundkörper als zweiter Durchgangskanal mindestens eine zweite Bohrung angeordnet ist, deren Durchgangsachse mit der Drehachse des Rührorgans einen zur Unterseite des Rührorgans sich öffnenden Winkel α mit 0° ≤ α < 90° einschließt.

5. Rührorgan nach einem der Ansprüche 3 oder 4, **dadurch gekennzeichnet, dass** der erste und der zweite Durchgangskanal sich kreuzen und/oder der erste und der zweite Durchgangskanal aufeinandertreffen und einen gemeinsamen Durchgangskanal bildend seitlich aus dem Rührorgan austreten.

6. Rührorgan nach einem der Ansprüche 1 bis 5, **dadurch gekennzeichnet, dass** zwei oder mehrere erste und/oder zweite Durchgangskanäle längs des Umfangs des Rührorgans gleichmäßig beabstandet zueinander angeordnet sind.

7. Rührorgan nach einem der Ansprüche 1 bis 6, **dadurch gekennzeichnet, dass** der Grundkörper im Querschnitt senkrecht zur Drehachse kreiszylindrisch, elliptisch, vieleckig, viereckig oder rechteckig ist.

8. Rührorgan nach einem der Ansprüche 1 bis 7, **dadurch gekennzeichnet, dass** der Grundkörper oval, eiförmig oder quaderförmig ist.

9. Rührorgan nach einem der Ansprüche 1 bis 8, **dadurch gekennzeichnet, dass** der Grundkörper längs seines Umfangsrandes in Drehrichtung Ausnehmungen aufweist.

10. Vorrichtung zur Kultivierung von Zellen in Flüssigkeitssäulen im Milliliter-Maßstab mit einem Behälter zur Aufnahme einer flüssigen Kultursuspension der Zellen,
einem Rührorgan zum Rühren der Kultursuspension in dem Behälter um eine Rührachse,
**dadurch gekennzeichnet, dass**
der Behälter und/oder das Rührorgan derart ausgebildet sind, dass die Strömungsgeschwindigkeit sich längs einer Stromlinie oder Bahnlinie um die Rührachse örtlich und/oder zeitlich ändert, und dass das Rührorgan ein Rührorgan nach einem der vorhergehenden Ansprüche ist.

11. Vorrichtung nach dem vorhergehenden Anspruch, **dadurch gekennzeichnet, dass** der Behälter und/oder das Rührorgan derart ausgebildet sind, dass durch das Rührorgan eine von der Suspensionsoberfläche zum Boden des Behälters gerichtete Förderung der Kultursuspension erfolgt.

12. Vorrichtung nach dem vorhergehenden Anspruch, **dadurch gekennzeichnet, dass** die Förderung der Kultursuspension eine bezüglich der Rührachse axiale Komponente aufweist.

13. Vorrichtung nach einem der Ansprüche 10-12, **dadurch gekennzeichnet, dass** die die Kultursuspension umschließenden Innenwände des Behälters unterhalb, in und/oder oberhalb der Drehebene des Rührorgans eine nicht rotationssymetrische Form aufweist.

14. Vorrichtung nach einem der Ansprüche 10-13, **dadurch gekennzeichnet, dass** die die Kultursuspension umschließenden Innenwände des Behälters unterhalb, in und/oder oberhalb der Drehebene des Rührorgans ein Vieleck, vorzugsweise mit vier, fünf, sechs oder mehr Ecken bildet.

15. Vorrichtung nach einem der Ansprüche 10-14, **dadurch gekennzeichnet, dass** die Drehachse des Rührorgans außermittig bzw. exzentrisch bezüglich der die Kultursuspension umschließenden Innenwände des Behälters in dem Behälter angeordnet ist.

16. Vorrichtung nach einem der Ansprüche 10-15, **dadurch gekennzeichnet, dass** an der Innenwand des Behälters längs des Umlaufs des Rührorgans mindestens ein Strömungsbrecher angeordnet ist.

17. Vorrichtung nach dem vorhergehenden Anspruch, **dadurch gekennzeichnet, dass** ein, zwei, drei, vier oder mehr Strömungsbrecher längs der Innenwand des Behälters beabstandet, vorteilhafterweise gleichmäßig beabstandet, zueinander angeordnet sind.

18. Vorrichtung nach einem der Ansprüche 16 oder 17, **dadurch gekennzeichnet, dass** der bzw. die Strömungsbrecher unterhalb, in und/oder oberhalb der Drehebene des Rührorgans angeordnet ist bzw. sind.

19. Vorrichtung nach einem der Ansprüche 16 bis 18, **dadurch gekennzeichnet, dass** das Rührorgan und der Strömungsbrecher mit einem minimaler Spaltabstand zueinander von 0,05 mm bis 20 mm, vorzugsweise 0,1 mm bis 3 mm angeordnet sind.

20. Vorrichtung nach einem der Ansprüche 10-19, **dadurch gekennzeichnet, dass** der Behälter ein Kolben, ein Reagenzglas oder eine Kavität einer Mikrotiterplatte oder einer anderen mit Kavitäten versehenen Platte ist.

21. Vorrichtung nach einem der Ansprüche 10-20, **dadurch gekennzeichnet, dass** das Rührorgan magnetisch positioniert und/oder antreibbar ist.

22. Vorrichtung nach einem der Ansprüche 10-21, **dadurch gekennzeichnet, dass** das Rührorgan gelagert oder ungelagert ist.

23. Vorrichtung nach einem der Ansprüche 10-22, **dadurch gekennzeichnet, dass** das Rührorgan über eine Welle gelagert und gegebenenfalls über die Welle antreibbar ist.

24. Vorrichtung nach dem vorhergehenden Anspruch, **dadurch gekennzeichnet, dass** die Welle und der bzw. die Strömungsbrecher einstückig ausgebildet sind.

25. Vorrichtung nach einem der Ansprüche 15 bis 18, 22 und 23, **dadurch gekennzeichnet, dass** die Welle und der bzw. die Strömungsbrecher in den Behälter einsteckbar ist bzw. sind.

26. Vorrichtung nach einem der Ansprüche 22 bis 25, **dadurch gekennzeichnet, dass** die Welle von oben in den Behälter ragt.

27. Vorrichtung nach einem der Ansprüche 10-26, **dadurch gekennzeichnet, dass** die Welle an ihrem unteren Ende seitlich verdickt ist bzw. einen Flansch aufweist.

28. Vorrichtung nach einem der Ansprüche 22 bis 27, **dadurch gekennzeichnet, dass** die Welle als Vollmaterialwelle oder als Hohlrohr ausgebildet ist.

29. Vorrichtung nach einem der Ansprüche 10-28, **dadurch gekennzeichnet, dass** die Welle als Hohlrohr ausgebildet ist und an ihrem unten offenen Ende eine Düse angeordnet ist.

30. Anordnung zur parallelen, automatisierten Kultivierung von Zellen in Flüssigkeitssäulen im Millilitermaßstab
**gekennzeichnet durch**
mindestens eine Vorrichtung nach einem der Ansprüche 10-29.

31. Anordnung nach dem vorhergehenden Anspruch, **gekennzeichnet durch** einen Block, in dem eine der Anzahl der Behälter entsprechende Anzahl Kavitäten angeordnet sind, welche selbst Behälter darstellen oder zur Aufnahme von Behältern ausgebildet sind.

32. Anordnung nach dem vorhergehenden Anspruch, **dadurch gekennzeichnet, dass** die Kavitäten als Bohrungen mit einem Durchmesser entsprechend dem Außendurchmesser der Behälter ausgebildet sind.

33. Anordnung nach einem der Ansprüche 31 oder 32, **dadurch gekennzeichnet, dass** in dem Block Mittel zum Temperieren des Blocks, Mittel zum Antreiben der Magnetrührorgane und/oder eine Sterilgaszufuhr zu den Behältern angeordnet sind.

34. Anordnung nach einem der Ansprüche 30 bis 33, **dadurch gekennzeichnet, dass** die Behälter, die Anordnung und/oder der Block auf ihrer bzw. seiner Oberseite mittels einer Abdeckung steril verschlossen ist.

35. Anordnung nach dem vorhergehenden Anspruch, **dadurch gekennzeichnet, dass** die Abdeckung eine Öffnung zur Freisetzung von Gasen und als Zugang zum Innenraum des Behälters oder Blocks aufweist, die sich geradlinig und länglich von dem Innenraum zur Außenseite der Abdeckung erstreckt und diese für Gaskonvektion offen miteinander verbindet.

36. Anordnung nach dem vorhergehenden Anspruch, **dadurch gekennzeichnet, dass** im Innenraum ein höherer Gasdruck als auf der Außenseite der Abdeckung vorliegt.

37. Anordnung nach einem der Ansprüche 35 oder 36, **dadurch gekennzeichnet, dass** die Öffnung die Form einer Bohrung oder eines Röhrchens aufweist.

38. Anordnung nach einem der Ansprüche 35 bis 37, **dadurch gekennzeichnet, dass** die Öffnung eine lichte Weite aufweist, die das Einführen eines Beprobungsorgans oder eines Sensors, einer Pipettenspitze, einer Anstechkanüle, einer pH-Elektrode oder eines anderen Gegenstandes bzw. länglichen Gegenstandes in den Innenraum des Behälters oder Blocks ermöglicht.

39. Anordnung nach einem der Ansprüche 35 bis 38, **dadurch gekennzeichnet, dass** die Öffnung ein Röhrchen aus Metall oder Metalllegierungen, vorteilhafterweise aus Aluminium und/oder Silber ist.

40. Anordnung nach einem der Ansprüche 35 bis 39, **gekennzeichnet durch** Behältertrennelemente, die in auf die Behälter bzw. den Block aufgesetztem Zustand einzelne Behälter voneinander gasdicht und/oder flüssigkeitsdicht abtrennt.

41. Anordnung nach einem der Ansprüche 35 bis 40, **dadurch gekennzeichnet, dass** die Abdeckung eine sterile Gaszufuhr zu dem oder zu mehreren Behältern, für alle Behälter gemeinsam oder für mehrere oder jeden der Behälter einzeln aufweist.

42. Anordnung nach dem vorhergehenden Anspruch, **dadurch gekennzeichnet, dass** die sterile Gaszufuhr in die Abdeckung integrierte oder der Abdeckung benachbart angeordnete Gasverteilerstrukturen aufweist.

43. Anordnung nach dem vorhergehenden Anspruch, **dadurch gekennzeichnet, dass** in der Abdeckung Kanäle als Gasverteilerstrukturen angeordnet sind.

44. Anordnung nach dem vorhergehenden Anspruch, **dadurch gekennzeichnet, dass** die Kanäle zwischen dem Gaseinlass in die Gasverteilerstruktur und den Gasauslässen in die jeweiligen Behälter sämtlich eine gleiche Länge und/oder dieselbe Anzahl Knicke aufweisen.

45. Anordnung nach einem der Ansprüche 40 bis 44, **dadurch gekennzeichnet, dass** die Gaszufuhr mit einer Gaseinspeisung, gegebenenfalls über Sterilfilter und/oder Luftbefeuchter verbunden ist.

46. Anordnung nach einem der Ansprüche 30 bis 45, **dadurch gekennzeichnet, dass** die Abdeckung mindestens eine flächige Schicht bzw. ebene Platte aufweist, die die Öffnung des mindestens einen Behälters abdeckt.

47. Anordnung nach dem vorhergehenden Anspruch, **dadurch gekennzeichnet, dass** sie mindestens zwei parallel zueinander angeordnete flächige Schichten aufweist, zwischen denen die Gasverteilerstrukturen angeordnet sind.

48. Verfahren zur Kultivierung von Zellen in Flüssigkeitssäulen im Milliliter-Maßstab, wobei mindestens eine Zellsuspension in einem Behälter derart bewegt wird, dass die Zellsuspension eine Ringströmung ausbildet
**dadurch gekennzeichnet, dass**
die Strömungsgeschwindigkeit der Ringströmung sich längs einer Strömungslinie örtlich und/oder zeitlich ändert und das Verfahren mit einer Vorrichtung nach einem der Ansprüche 10 bis 29 durchgeführt wird.

49. Verfahren nach dem vorhergehenden Anspruch, **dadurch gekennzeichnet, dass** der Behälter und/oder das Rührorgan derart ausgebildet sind, eine von der Suspensionsoberfläche zum Boden des Behälters gerichtete Förderung der Kultursuspension erfolgt.

50. Verfahren nach einem der Ansprüche 48 oder 49, **dadurch gekennzeichnet, dass** die mindestens eine Zellsuspension in einer Vorrichtung oder Anordnung nach einem der vorhergehenden Ansprüche unter Rühren mit einem Rührorgan kultiviert wird.

51. Verfahren nach dem vorhergehenden Anspruch, **dadurch gekennzeichnet, dass** das Rührorgan mit 1 bis 4000 Umdrehungen pro Minute angetrieben wird.

## Claims

1. Mixing element having a basic body which, in operation, has an upper side and an underside,
***characterised in that**,*
in the basic body, at least one first through-channel is disposed, the first opening of which is situated at least partially on the underside of the basic body and the second opening of which is situated on the upper side and/or laterally on the basic body, the mixing element being shaped such that the flow velocity along a streamline or a path line around the mixing axis varies spatially and/or temporally.

2. Mixing element according to the preceding claim, **characterised in that**, in the basic body, at least one first boring is disposed as first through-channel, the passage axis of which boring includes an angle α with 0° ≤ α < 90° with the rotational axis of the mixing element, said angle opening to the upper side of the mixing element.

3. Mixing element according to one of claims 1 or 2, **characterised in that**, in the basic body, at least one further second through-channel is disposed, the first opening of which is situated at least partially on the upper side of the basic body and the second opening of which is situated on the underside and/or laterally on the basic body.

4. Mixing element according to the preceding claim, **characterised in that**, in the basic body, at least one second boring is disposed as second through-channel, the passage axis of which boring includes an angle α with 0° ≤ α < 90° with the rotational axis of the mixing element, said angle opening to the underside of the mixing element.

5. Mixing element according to one of claims 3 or 4, **characterised in that** the first and the second through-channel intersect and/or the first and the second through-channel meet each other and, forming a common through-channel, emerge laterally from the mixing element.

6. Mixing element according to one of the claims 1 to 5, **characterised in that** two or more first and/or second through-channels are disposed along the circumference of the mixing element at a uniform spacing relative to each other.

7. Mixing element according to one of the claims 1 to 6, **characterised in that** the basic body, in the cross-section perpendicular to the rotational axis, is circular cylindrical, elliptical, polygonal, quadrangular or rectangular.

8. Mixing element according to one of the claims 1 to 7, **characterised in that** the basic body is oval, egg-shaped or cuboid.

9. Mixing element according to one of the claims 1 to 8, **characterised in that** the basic body has recesses along its circumferential edge in the rotational direction.

10. Device for cultivating cells in liquid columns on a millilitre scale, having a container for receiving a liquid culture suspension of the cells,
a mixing element for mixing the culture suspension in the container about a mixing axis,
***characterised in that***
the container and/or the mixing element are configured in such a manner that the flow velocity is modified locally and/or temporally along a streamline or flow line about the mixing axis, and that the mixing element is a mixing element according to one of the proceeding claims.

11. Device according to the preceding claim, **characterised in that** the container and/or the mixing element are configured in such a manner that, by means of the mixing element, conveyance of the culture suspension which is directed from the suspension surface to the base of the container is effected.

12. Device according to the preceding claim, **characterised in that** the conveyance of the culture suspension has a component which is axial with respect to the mixing axis.

13. Device according to one of the claims 10-12, **characterised in that** the inner walls of the container which enclose the culture suspension have, below, in and/or above the rotational plane of the mixing element, a non-rotationally symmetrical form.

14. Device according to one of the claims 10-13, **characterised in that** the inner walls of the container which enclose the culture suspension form, below, in and/or above the rotational plane of the mixing element, a polygon, preferably with four, five, six or more corners.

15. Device according to one of the claims 10-14, **characterised in that** the rotational axis of the mixing element is disposed off-centre or eccentically in the container with respect to the inner walls of the container which enclose the culture suspension.

16. Device according to one of the claims 10-15, **characterised in that** at least one flow breaker is disposed on the inner wall of the container along the circumference of the mixing element.

17. Device according to the preceding claim, **characterised in that** one, two, three, four or more flow breakers are disposed along the inner wall of the container, at a spacing, advantageously at a uniform spacing, relative to each other.

18. Device according to one of the claims 16 or 17, **characterised in that** the flow breaker or breakers is or are disposed below, in and/or above the rotational plane of the mixing element.

19. Device according to one of the claims 16 to 18, **characterised in that** the mixing element and the flow breaker are disposed at a minimum gap spacing relative to each other of 0.05 mm to 20 mm, preferably 0.1 mm to 3 mm.

20. Device according to one of the claims 10-19, **characterised in that** the container is a flask, a reagent glass or a cavity of a microtitre plate or of another plate which is provided with cavities.

21. Device according to one of the claims 10-20, **characterised in that** the mixing element is magnetically positioned and/or driveable.

22. Device according to one of the claims 10-21, **characterised in that** the mixing element is mounted or not mounted.

23. Device according to one of the claims 10-22, **characterised in that** the mixing element is mounted via a shaft and if necessary is driveable via the shaft.

24. Device according to the preceding claim, **characterised in that** the shaft and the flow breaker or breakers are configured in one piece.

25. Device according to one of the claims 15 to 18, 22 and 23, **characterised in that** the shaft and the flow breaker or breakers is or are insertable into the container.

26. Device according to one of the claims 22 to 25, **characterised in that** the shaft protrudes into the container from above.

27. Device according to one of the claims 10-26, **characterised in that** the shaft is enlarged at its lower end or has a flange.

28. Device according to one of the claims 22 to 27, **characterised in that** the shaft is configured as a solid material shaft or as a hollow pipe.

29. Device according to one of the claims 10-28, **characterised in that** the shaft is configured as a hollow pipe and a nozzle is disposed on its end which is open at the bottom.

30. Arrangement for parallel, automated cultivation of cells in liquid columns on a millilitre scale,
**characterised by**
at least one device or mixing element according to one of the claims 10-29.

31. Arrangement according to the preceding claim, **characterised by** a block, in which a number of cavities, which corresponds to the number of containers, are disposed, which represent containers themselves or are configured for receiving containers.

32. Arrangement according to the preceding claim, **characterised in that** the cavities are configured as borings with a diameter corresponding to the external diameter of the containers.

33. Arrangement according to one of the two claims 31 or 32, **characterised in that** means for moderating the temperature of the block, means for driving the magnetic mixing elements and/or a sterile gas supply to the containers are disposed in the block.

34. Arrangement according to one of the claims 30 to 33, **characterised in that** the containers, the arrangement and/or the block are sealed in a sterile manner on their or its upper side by means of a cover.

35. Arrangement according to the preceding claim, **characterised in that** the cover has an opening for the release of gases and as access to the interior of the container or block, which opening extends in a straight line and longitudinally from the interior to the outer side of the cover and connects these together in an open manner for gas convection.

36. Arrangement according to the preceding claim, **characterised in that**, in the interior, a higher gas pressure is present than on the outer side of the cover.

37. Arrangement according to one of the claims 35 or 36, **characterised in that** the opening has the form of a boring or of a tube.

38. Arrangement according to one of the 35 to 37 claims, **characterised in that** the opening has a clearance width, which makes possible the introduction of a sampling element or of a sensor, a pipette tip, a piercing cannula, a pH electrode or another object or elongated object into the interior of the container or block.

39. Arrangement according to one of the claims 35 to 38, **characterised in that** the opening is a tube made of metal or metal alloys, advantageously made of aluminium and/or silver.

40. Arrangement according to one of the claims 35 to 39, **characterised by** container separating elements which, when in the state set upon the containers or the block, separate individual containers from each other in a gas- and/or liquid-impermeable manner.

41. Arrangement according to one of the claims 35 to 40, **characterised in that** the cover has a sterile gas supply to the one or a plurality of containers, for all the containers together or for a plurality or each of the containers separately.

42. Arrangement according to the preceding claim, **characterised in that** the sterile gas supply has gas distributor structures which are integrated in the cover or disposed adjacent to the cover.

43. Arrangement according to the preceding claim, **characterised in that** channels are disposed in the cover as gas distributor structures.

44. Arrangement according to the preceding claim, **characterised in that** the channels between the gas inlet into the gas distributor structure and the gas outlets into the respective containers all have the same length and/or the same number of bends.

45. Arrangement according to one of the claims 40 to 44, **characterised in that** the gas supply is connected to a gas feed, if necessary via sterile filters and/or air humidifiers.

46. Arrangement according to one of the claims 30 to 45, **characterised in that** the cover has at least one planar layer or flat plate, which covers the opening of the at least one container.

47. Arrangement according to the preceding claim, **characterised in that** it has at least two planar layers which are disposed parallel to each other and between which the gas distributor structures are disposed.

48. Method for cultivating cells in liquid columns on a millilitre scale, at least one cell suspension being moved in a container in such a manner that the cell suspension forms an annular flow, ***characterised in that***
the flow velocity of the annular flow is modified locally and/or temporally along a streamline, and the method is carried out with a device according to claims 10 to 29.

49. Method according to the preceding claim, **characterised in that** the container and/or the mixing element are configured in such a manner that conveyance of the culture suspension which is directed from the suspension surface to the base of the container is effected.

50. Method according to one of the claims 48 or 49, **characterised in that** the at least one cell suspension is cultivated in a device or arrangement according to one of the preceding claims by mixing with a mixing element.

51. Method according to the preceding claim, **characterised in that** the mixing element is driven with 1 to 4000 revolutions per minute.

## Revendications

1. Organe d'agitation avec un corps de base, qui comprend, lors du fonctionnement, un côté supérieur et un côté inférieur, **caractérisé en ce que**, dans le corps de base, se trouve au moins un canal de passage, dont la première ouverture se trouve au moins partiellement sur le côté inférieur du corps de base et dont la deuxième ouverture se trouve sur le côté supérieur et/ou sur latéralement sur le corps de base, l'organe d'agitation étant conçu de façon à ce que la vitesse d'écoulement varie localement et/ou dans le temps le long d'une ligne d'écoulement ou d'une trajectoire autour de l'axe d'agitation.

2. Organe d'agitation selon la revendication précédente, **caractérisé en ce que**, dans le corps de base, en tant que premier canal de passage, se trouve au moins un premier alésage dont l'axe de passage forme, avec l'axe de rotation de l'organe d'agitation, un angle α s'ouvrant vers le côté supérieur de l'organe d'agitation, avec 0° ≤ α < 90°.

3. Organe d'agitation selon l'une des revendications 1 ou 2, **caractérisé en ce que**, dans le corps de base, se trouve au moins un deuxième canal de passage dont la première ouverture se trouve au moins partiellement sur le côté supérieur du corps de base et dont la deuxième ouverture se trouve sur le côté inférieur et/ou latéralement sur le corps de base.

4. Organe d'agitation selon la revendication précédente, **caractérisé en ce que**, dans le corps de base, en tant que deuxième canal de passage, se trouve au moins un deuxième alésage dont l'axe de passage forme, avec l'axe de rotation de l'organe d'agitation, un angle α s'ouvrant vers le côté inférieur de l'organe d'agitation, avec 0° ≤ α < 90°.

5. Organe d'agitation selon l'une des revendications 3 ou 4, **caractérisé en ce que** le premier et le deuxième canaux de passage se croisent et/ou le premier et le deuxième canaux de passage se rejoignent et sortent latéralement de l'organe d'agitation en formant un canal de passage commun.

6. Organe d'agitation selon l'une des revendications 1 à 5, **caractérisé en ce que** deux premiers et/ou deuxièmes canaux de passage ou plus sont disposés de manière écartée uniformément entre eux le long de la périphérie de l'organe d'agitation.

7. Organe d'agitation selon l'une des revendications 1 à 6, **caractérisé en ce que** le corps de base présente une section perpendiculaire à l'axe de rotation de forme cylindrique, elliptique, polygonale, quadrangulaire ou rectangulaire.

8. Organe d'agitation selon l'une des revendications 1 à 7, **caractérisé en ce que** le corps de base présente une forme ovale, ovoïde ou parallélépipédique.

9. Organe d'agitation selon l'une des revendications 1 à 8, **caractérisé en ce que** le corps de base présente des évidements le long du bord périphérique dans le sens de rotation.

10. Dispositif de culture de cellules dans des colonnes de liquide de l'ordre du millimètre avec un récipient pour le logement d'une suspension de culture liquide des cellules,
un organe d'agitation pour l'agitation de la suspension de culture dans le récipient autour d'un axe d'agitation,
**caractérisé en ce que**
le récipient et/ou l'organe d'agitation est conçu de façon à ce que la vitesse d'écoulement varie localement et/ou dans le temps le long d'une ligne d'écoulement ou d'une trajectoire autour de l'axe d'agitation,
et **en ce que** l'organe d'agitation est un organe d'agitation selon l'une des revendications précédentes.

11. Dispositif selon la revendication précédente, **caractérisé en ce que** le récipient et/ou l'organe d'agitation sont conçus de façon à ce que l'organe d'agitation permette un transport de la suspension de culture dirigée de la surface de la suspension vers le fond du récipient.

12. Dispositif selon la revendication précédente, **caractérisé en ce que** le transport de la suspension de culture présente une composante axiale par rapport à l'axe d'agitation.

13. Dispositif selon l'une des revendications 10 à 12, **caractérisé en ce que** les parois internes du récipient entourant la suspension de culture présentent, en dessous, dans et/ou au-dessus du plan de rotation de l'organe d'agitation, une forme sans symétrie de rotation.

14. Dispositif selon l'une des revendications 10 à 13, **caractérisé en ce que** les parois internes du récipient entourant la suspension de culture forment, en dessous, dans et/ou au-dessus du plan de rotation de l'organe d'agitation, un polygone, de préférence avec quatre, cinq, six côtés ou plus.

15. Dispositif selon l'une des revendications 10 à 14, **caractérisé en ce que** l'axe de rotation de l'organe d'agitation est disposé, dans le récipient, de manière excentrée par rapport aux parois internes du récipient entourant la suspension de culture.

16. Dispositif selon l'une des revendications 10 à 15, **caractérisé en ce que**, sur la paroi interne du récipient, le long de la périphérie de l'organe d'agitation, se trouve au moins un brise-flux.

17. Dispositif selon la revendication précédente, **caractérisé en ce qu'**un, deux, trois, quatre brise-flux ou plus sont disposés de manière écartée entre eux, de préférence écartées de manière uniforme, le long de la paroi interne du récipient.

18. Dispositif selon l'une des revendications 16 ou 17, **caractérisé en ce que** le ou les brise-flux sont disposés en dessous et,selon le cas, dans et/ou au-dessus du plan de rotation de l'organe d'agitation.

19. Dispositif selon l'une des revendications 16 à 18, **caractérisé en ce que** l'organe d'agitation et le brise-flux sont disposés, avec un interstice minimal entre eux de 0,05 mm à 20 mm, de préférence de 0,1 mm à 3 mm.

20. Dispositif selon l'une des revendications 10 à 19, **caractérisé en ce que** le récipient est un piston, une éprouvette ou une cavité d'une plaque de micro-titrage ou d'une autre plaque munie de cavités.

21. Dispositif selon l'une des revendications 10 à 20, **caractérisé en ce que** l'organe d'agitation est positionné et/ou peut être entraîné de manière magnétique.

22. Dispositif selon l'une des revendications 10 à 21, **caractérisé en ce que** l'organe d'agitation est muni d'un palier ou non.

23. Dispositif selon l'une des revendications 10 à 22, **caractérisé en ce que** l'organe d'agitation est logé à l'aide d'un arbre et le cas échéant il peut être entraîné par l'intermédiaire de l'arbre.

24. Dispositif selon la revendication précédente, **caractérisé en ce que** l'arbre et le ou les brise-flux sont conçus d'une seule pièce.

25. Dispositif selon l'une des revendications 15 à 18, 22 et 23, **caractérisé en ce que** l'arbre et le ou les brise-flux peuvent être insérés dans le récipient.

26. Dispositif selon l'une des revendications 22 à 25, **caractérisé en ce que** l'arbre dépasse dans le récipient par le haut.

27. Dispositif selon l'une des revendications 10 à 26, **caractérisé en ce que** l'arbre est épaissi latéralement ou présente une bride au niveau de son extrémité inférieure.

28. Dispositif selon l'une des revendications 22 à 27, **caractérisé en ce que** l'arbre est conçu comme un arbre massif ou comme un tube creux.

29. Dispositif selon l'une des revendications 10 à 28, **caractérisé en ce que** l'arbre est conçu comme un tube creux et une buse est disposée au niveau de son extrémité ouverte vers le bas.

30. Dispositif pour la culture automatisée de cellules dans des colonnes de liquide de l'ordre du millimètre
**caractérisé par**
au moins un dispositif selon l'une des revendications 10 à 29.

31. Dispositif selon la revendication précédente, **caractérisé par** un bloc, dans lequel est disposé un nombre de cavités correspondant au nombre de récipients, qui constituent elles-mêmes des récipients ou sont conçues pour le logement de récipients.

32. Dispositif selon la revendication précédente, **caractérisé en ce que** les cavités sont conçues comme des alésages avec un diamètre correspondant au diamètre extérieur des récipients.

33. Dispositif selon l'une des revendications 31 ou 32, **caractérisé en ce que**, dans le bloc, sont disposés des moyens pour la régulation en température du bloc, des moyens pour l'entraînement des organes d'agitation magnétiques et/ou une alimentation en gaz stérile vers les récipients.

34. Dispositif selon l'une des revendications 30 à 33, **caractérisé en ce que** les récipients, le dispositif et/ou le bloc sont fermés, au niveau de leur côté supérieur, au moyen d'un couvercle.

35. Dispositif selon la revendication précédente, **caractérisé en ce que** le couvercle présente une ouverture pour la libération de gaz et en tant qu'entrée vers l'intérieur du récipient ou du bloc, qui s'étend en ligne droite et de manière oblongue de l'intérieur vers l'extérieur du couvercle et relie ceux-ci entre eux pour une convection de gaz.

36. Dispositif selon la revendication précédente, **caractérisé en ce que**, sur le côté intérieur du couvercle, règne une pression de gaz supérieure à celle qui règne du côté extérieur du couvercle.

37. Dispositif selon l'une des revendications 35 ou 36, **caractérisé en ce que** l'ouverture présente la forme d'un alésage ou d'un petit tube.

38. Dispositif selon l'une des revendications 35 à 37, **caractérisé en ce que** l'ouverture présente un diamètre intérieur qui permet l'introduction d'un organe d'échantillonnage, d'une canule de perçage, d'une électrode à pH ou d'un autre objet ou d'un objet oblong à l'intérieur du récipient ou du bloc.

39. Dispositif selon l'une des revendications 35 à 38, **caractérisé en ce que** l'ouverture est un petit tube en métal en alliage métallique, de préférence en aluminium et/ou en argent.

40. Dispositif selon l'une des revendications 35 à 39, **caractérisé par** des éléments de séparation de récipients qui sépare, lorsqu'il est posé sur les récipients ou sur le bloc, les différents récipients entre eux de manière étanche aux gaz et/ou étanche aux liquides.

41. Dispositif selon l'une des revendications 35 à 40, **caractérisé en ce que** le couvercle comprend une alimentation en gaz stérile vers le ou les récipients, commune à tous les récipients ou pour plusieurs récipients ou individuellement pour chacun des récipients.

42. Dispositif selon la revendication précédente, **caractérisé en ce que** l'alimentation en gaz stérile comprend des structures de distribution de gaz intégrées ou disposées à proximité du couvercle.

43. Dispositif selon la revendication précédente, **caractérisé en ce que**, dans le couvercle se trouvent des canaux en tant que structure de distribution de gaz.

44. Dispositif selon la revendication précédente, **caractérisé en ce que** les canaux présentent, entre l'entrée de gaz dans la structure de distribution de gaz et les sorties de gaz dans les différents récipients, la même longueur et/ou le même nombre de coudes.

45. Dispositif selon l'une des revendications 40 à 44, **caractérisé en ce que** l'alimentation en gaz est reliée avec une injection de gaz, le cas échéant par l'intermédiaire d'un filtre stérile et/ou d'un humidificateur d'air.

46. Dispositif selon l'une des revendications 30 à 45, **caractérisé en ce que** le couvercle comprend au moins une couche plane ou une plaque plate qui recouvre l'ouverture de l'au moins un récipient.

47. Dispositif selon la revendication précédente, **caractérisé en ce qu'**il comprend au moins deux couches planes parallèles entre elles, entre lesquelles se trouvent les structures de distribution de gaz.

48. Procédé de culture de cellules dans des colonnes de liquide de l'ordre du millimètre, au moins une suspension de cellules étant mise en mouvement dans un récipient de façon à ce que la suspension de cellules forme un écoulement annulaire, **caractérisé en ce que** la vitesse d'écoulement de l'écoulement annulaire varie localement et/ou dans le temps le long de la ligne d'écoulement et le procédé est réalisé avec un dispositif selon l'une des revendications 10 à 29.

49. Procédé selon la revendication précédente, **caractérisé en ce que** le récipient et/ou l'organe d'agitation sont conçus de façon à permettre un transport de la suspension de culture dirigé de la surface de la suspension vers le fond du récipient.

50. Procédé selon l'une des revendications 48 ou 49, **caractérisé en ce que** l'au moins une suspension de cellules est cultivée dans un dispositif selon l'une des revendications précédentes en agitant avec un organe d'agitation.

51. Procédé selon la revendication précédente, **caractérisé en ce que** l'organe d'agitation est entraîné de 1 à 4000 tours par minute.
